# EUROPEAN PATENT SPECIFICATION

(11) **EP 4 571 756 B1**
(45) Date of publication and mention of the grant of the patent: **23.09.2026**
(21) Application number: 23922383.7
(22) Date of filing: 08.11.2023
(51) Int. Cl.: G16B 40/20, G06F 18/213

(54) **ELECTROENCEPHALOGRAM ANALYSIS MODEL TRAINING METHOD AND APPARATUS, COMPUTER DEVICE, COMPUTER-READABLE STORAGE MEDIUM AND COMPUTER PROGRAM PRODUCT**
TRAININGSVERFAHREN UND -VORRICHTUNG FÜR ELEKTROENZEPHALOGRAMMANALYSEMODELL, COMPUTERVORRICHTUNG, COMPUTERLESBARES SPEICHERMEDIUM UND COMPUTERPROGRAMMPRODUKT
PROCÉDÉ ET APPAREIL D'ENTRAÎNEMENT DE MODÈLE D'ANALYSE D'ÉLECTROENCÉPHALOGRAMME, DISPOSITIF INFORMATIQUE, SUPPORT DE STOCKAGE LISIBLE PAR ORDINATEUR ET PRODUIT PROGRAMME D'ORDINATEUR

(30) Priority: 14.02.2023 CN 202310149984
(43) Date of publication of application: 18.06.2025
(73) Proprietor: Tencent Technology (Shenzhen) Company Limited, Shenzhen, Guangdong 518057 (CN)
(72) Inventor: ZHOU, Yanning, Shenzhen, Guangdong 518057 (CN); XIAO, Kaiwen, Shenzhen, Guangdong 518057 (CN); YE, Jingwen, Shenzhen, Guangdong 518057 (CN); SHEN, Tian, Shenzhen, Guangdong 518057 (CN); LIU, Sibo, Shenzhen, Guangdong 518057 (CN); ZHU, Yiqin, Shenzhen, Guangdong 518057 (CN); WANG, Ziyuan, Shenzhen, Guangdong 518057 (CN); HAN, Xiao, Shenzhen, Guangdong 518057 (CN)
(74) Representative: Nederlandsch Octrooibureau
(86) International application number: PCT/CN2023/130339
(87) International publication number: WO 2024/169267

(56) References cited:
- CN-A- 113 842 152
- CN-A- 115 590 515
- CN-A- 116 955 983
- US-B1- 11 152 123
- LI RUI REALEE@SJTU EDU CN ET AL: "A Multi-view Spectral-Spatial-Temporal Masked Autoencoder for Decoding Emotions with Self-supervised Learning", PROCEEDINGS OF THE 30TH ACM INTERNATIONAL CONFERENCE ON MULTIMEDIA, ACMPUB27, NEW YORK, NY, USA, 10 October 2022 (2022-10-10), pages 6 - 14, XP058896163, ISBN: 978-1-4503-9036-1, DOI: 10.1145/3503161.3548243
- GEXIN HUANG ET AL: "Electromagnetic Source Imaging via a Data-Synthesis-Based Convolutional Encoder-Decoder Network", ARXIV.ORG, CORNELL UNIVERSITY LIBRARY, 201 OLIN LIBRARY CORNELL UNIVERSITY ITHACA, NY 14853, 13 July 2022 (2022-07-13), XP091269504

## Description

### FIELD

The present disclosure relates to the technical field of computers, and in particular, to a method and apparatus for training an electroencephalogram signal analysis model, a computer device, a computer-readable storage medium, and a computer program product.

### BACKGROUND

A brain-computer interface is a technology in which a direct communication pathway is established between a human or animal brain and an external device to implement information exchange between the brain and the device. The brain-computer interface detects and identifies activation effects of different encephalic regions by analyzing an electroencephalogram signal to determine a user intention, thereby implementing direct communication and control between the human brain and the external device. The brain-computer interface has a wide application prospect in fields such as game entertainment, industrial processes, and medical engineering. However, due to the complexity and instability of electroencephalogram signals, electroencephalogram signals from a same subject in different environments or from multiple subjects in the same environment vary significantly, which results in limited reusability of training data, and reduced model performance. Therefore, how to alleviate the difference between the electroencephalogram signals to improve the model performance is a technical problem that needs to be solved.

In a related art, a problem of reduced model performance caused by the difference in the training data is usually alleviated by an adversarial training method. However, implementation of this solution relies on a large amount of real electroencephalogram signal data, the model has significant limitations, and the model can achieve good performance only in a specific application scenario. Therefore, there is an urgent need for a new method for training an electroencephalogram signal analysis model to train an electroencephalogram signal analysis model that can accurately analyze in a plurality of application scenarios.

LI RUI REALEE(S)SJTU EDU CN ET AL: "A Multi-view Spectral-Spatial-Temporal Masked Autoencoder for Decoding Emotions with Self-supervised Learning", PROCEEDINGS OF THE 30TH ACM INTERNATIONAL CONFERENCE ON MULTIMEDIA (XP058896163) provides a Multiview Spectral-Spatial-Temporal Masked Autoencoder based on a multi-view CNN-Transformer hybrid structure.

GEXIN HUANG ET AL: "Electromagnetic Source Imaging via a Data-Synthesis-Based Convolutional Encoder-Decoder Network", ARXIV.ORG, CORNELL UNIVERSITY LIBRARY, 201 OLIN LIBRARY CORNELL UNIVERSITY ITHACA, NY 14853 (XP091269504) provides a data-synthesized spatio-temporally convolutional encoder-decoder network method for ESI.

CN 115590515 A provides an emotion recognition method and system based on generative self-supervised learning and electroencephalogram signals.

### SUMMARY

The invention is set out in the appended set of claims. Embodiments of the present disclosure provide a method and apparatus for training an electroencephalogram signal analysis model, a computer device, a computer-readable storage medium, and a computer program product, which can enable a trained electroencephalogram signal analysis model to have a universal decoding capability for an electroencephalogram signal, can accurately decode electroencephalogram information in a real electroencephalogram signal, and can be widely applied to various downstream task scenarios. Technical solutions are as follows:

The embodiments of the present disclosure provide a method for training an electroencephalogram signal analysis model, applied to a computer device. The method includes:
masking each of an embedding vector of a simulated source signal and an embedding vector of a simulated electroencephalogram signal to obtain a mask vector of the simulated source signal and a mask vector of the simulated electroencephalogram signal, where the simulated source signal is configured for simulating an electrophysiological signal generated inside a brain of a target object, and the simulated electroencephalogram signal is configured for simulating an electrical signal measured from a brain scalp of the target object based on a sensor;
performing signal reconstruction on the simulated source signal and the simulated electroencephalogram signal based on the mask vector of the simulated source signal and the mask vector of the simulated electroencephalogram signal to obtain a reconstructed source signal and a reconstructed electroencephalogram signal, where the signal reconstruction includes encoding and decoding; and
training an electroencephalogram signal analysis model based on a difference between the simulated source signal and the reconstructed source signal and a difference between the simulated electroencephalogram signal and the reconstructed electroencephalogram signal.

The embodiments of the present disclosure provide an apparatus for training an electroencephalogram signal analysis model. The apparatus includes:
a first obtaining module, configured to obtain a first embedding vector of a simulated source signal and a second embedding vector of a simulated electroencephalogram signal, where the simulated source signal is configured for simulating an electrophysiological signal generated inside a brain of a target object, and the simulated electroencephalogram signal is configured for simulating an electrical signal measured from a brain scalp of the target object based on a sensor;
a mask module, configured to mask each of the first embedding vector and the second embedding vector to correspondingly obtain a first mask vector of the simulated source signal and a second mask vector of the simulated electroencephalogram signal;
a reconstruction module, configured to: perform signal reconstruction on the simulated source signal based on the first mask vector to obtain a reconstructed source signal, and perform signal reconstruction on the simulated electroencephalogram signal based on the second mask vector to obtain a reconstructed electroencephalogram signal, the signal reconstruction including encoding and decoding; and
a first training module, configured to train an electroencephalogram signal analysis model based on a difference between the simulated source signal and the reconstructed source signal and a difference between the simulated electroencephalogram signal and the reconstructed electroencephalogram signal.

The embodiments of the present disclosure provide a computer device. The computer device includes a processor and a memory, the memory is configured to store at least one computer program, and the at least one computer program is loaded and executed by the processor to perform the method for training an electroencephalogram signal analysis model in the embodiments of the present disclosure.

The embodiments of the present disclosure provide a computer-readable storage medium. The computer-readable storage medium stores at least one program, and the at least one program is loaded and executed by a processor to implement the method for training an electroencephalogram signal analysis model in the embodiments of the present disclosure.

The embodiments of the present disclosure provide a computer program product, including a computer program. The computer program implements, when executed by a processor, the method for training an electroencephalogram signal analysis model in the embodiments of the present disclosure.

The embodiments of the present disclosure provide a method for training an electroencephalogram signal analysis model. A first embedding vector of a simulated source signal and a second embedding vector of a simulated electroencephalogram signal are respectively masked, so that a masked first embedding vector (that is, a first mask vector) and a masked second embedding vector (that is, a second mask vector) can be obtained. In this way, randomness of input data can be increased. Then, the first mask vector and the second mask vector are respectively encoded and decoded, so that a reconstructed source signal corresponding to the simulated source signal and a reconstructed electroencephalogram signal corresponding to the simulated electroencephalogram signal can be reconstructed. Further, the electroencephalogram signal analysis model is trained based on a difference between the simulated source signal and the reconstructed source signal and a difference between the simulated electroencephalogram signal and the reconstructed electroencephalogram signal, whereby a trained electroencephalogram signal analysis model can have a universal decoding capability for electroencephalogram signals collected in various scenarios, accordingly can accurately decode electroencephalogram information in a real electroencephalogram signal, and can be widely applied to various downstream task scenarios.

### BRIEF DESCRIPTION OF THE DRAWINGS

To describe technical solutions of embodiments of the present disclosure more clearly, the following briefly introduces accompanying drawings required for describing the embodiments. Apparently, the accompanying drawings in the following description are merely some embodiments of the present disclosure, and a person of ordinary skill in the art may obtain other accompanying drawings from these accompanying drawings without creative efforts.
FIG. 1 is a schematic diagram of an implementation environment of a method for training an electroencephalogram signal analysis model according to an embodiment of the present disclosure.
FIG. 2 is a flowchart of a method for training an electroencephalogram signal analysis model according to an embodiment of the present disclosure.
FIG. 3 is a flowchart of another method for training an electroencephalogram signal analysis model according to an embodiment of the present disclosure.
FIG. 4 is a schematic diagram of an encephalic region distribution according to an embodiment of the present disclosure.
FIG. 5 is a schematic structural diagram of an embedding module according to an embodiment of the present disclosure.
FIG. 6 is a schematic structural diagram of a deconvolution module according to an embodiment of the present disclosure.
FIG. 7 is a schematic structural diagram of an electroencephalogram signal analysis model according to an embodiment of the present disclosure.
FIG. 8 is a schematic diagram of a training process of an electroencephalogram signal analysis model according to an embodiment of the present disclosure.
FIG. 9 is a block diagram of an apparatus for training an electroencephalogram signal analysis model according to an embodiment of the present disclosure.
FIG. 10 is a block diagram of another apparatus for training an electroencephalogram signal analysis model according to an embodiment of the present disclosure.
FIG. 11 is a structural block diagram of a terminal according to an embodiment of the present disclosure.
FIG. 12 is a schematic structural diagram of a server according to an embodiment of the present disclosure.

### DESCRIPTION OF EMBODIMENTS

To make objectives, technical solutions, and advantages of the present disclosure clearer, the following further describes implementations of the present disclosure in detail with reference to the accompanying drawings.

Terms "first", "second", and the like in the present disclosure are configured for distinguishing between the same or similar items with basically the same effects and functions. "First", "second", and "n^{th}" do not have a logical or chronological dependency on each other or limit a quantity and order of execution.

In the present disclosure, the term "at least one" refers to one or more, and "a plurality of" refers to two or more.

Information (including, but not limited to, user device information, user personal information, or the like), data (including, but not limited to, data configured for analysis, stored data, presented data, or the like), and signals involved in the present disclosure are all authorized by a user or fully authorized by various parties, and collection, use, and processing of related data need to comply with relevant laws, regulations, and standards of relevant regions. For example, a source signal, a simulated source signal, an electroencephalogram signal, and a simulated electroencephalogram signal involved in the present disclosure are obtained under full authorization.

Terms involved in the present disclosure are explained below.
(1) Artificial intelligence (AI): AI involves a theory, a method, a technology, and an application system that use a digital computer or a machine controlled by the digital computer to simulate, extend, and expand human intelligence, perceive an environment, obtain knowledge, and use knowledge to obtain an optimal result. In other words, AI is a comprehensive technology in computer science and attempts to understand the essence of intelligence and produce a new intelligent machine that can react in a manner similar to human intelligence. AI is to study the design principles and implementation methods of various intelligent machines, to enable the machines to have the functions of perception, reasoning, and decision-making.
(2) The AI technology is a comprehensive discipline, and relates to a wide range of fields including both hardware-level technologies and software-level technologies. The basic AI technologies generally include technologies such as a sensor, a dedicated AI chip, cloud computing, distributed storage, a big data processing technology, an operating/interaction system, and electromechanical integration. AI software technologies mainly include several major directions such as a computer vision (CV) technology, a speech processing technology, a natural language processing technology and machine learning/deep learning, autonomous driving, and smart transportation.
(3) Machine learning (ML): ML is a multi-field interdisciplinary technology, and relates to a plurality of disciplines such as a probability theory, statistics, an approximation theory, convex analysis, and an algorithm complexity theory. ML specializes in studying how a computer simulates or implements a human learning behavior to obtain new knowledge or skills, and reorganize an existing knowledge structure, so as to keep improving performance of the computer. ML is the core of AI, is a basic way to make the computer intelligent, and is applied to various fields of AI. ML and deep learning generally include technologies such as an artificial neural network, a belief network, reinforcement learning, transfer learning, inductive learning, and learning from demonstrations.
(4) Brain-computer interface (BCI): BCI refers to that a direct connection is created between a human or an animal brain and an external device, so that direct exchange between the brain and the device is implemented. The generalized brain-computer interface includes, but is not limited to, a brain signal, a muscle signal, or another signal, and finally aims to implement interaction of virtual and physical combination between the virtual world and the real world.
(5) An electroencephalography (EEG): EEG is a non-invasively used method for measuring electrical activities of a brain. An electrode needs to be placed on a scalp to record a voltage potential generated by a current within and around a neuron.
(6) Motor imagery (MI): When it is imagined that limbs (or muscles) of a person move but there is no actual movement output, a particular encephalic region of the person is still activated. Activation effects of different encephalic regions are detected and identified by analyzing an electroencephalogram signal to determine a user intention, thereby implementing direct communication and control between the human brain and the external device. Currently, common motor imagery positions are: left and right, a right hand, two legs and a tongue.
(7) Convolutional neural network (CNN): The CNN is a feedforward neural network, which includes several convolutional layers and pooling layers.
(8) Transformer: The transformer is a deep learning model, and uses a self-attention mechanism to perform difference weighting on significance of each part of input data. The transformer is mainly applied to the fields of natural language processing and computer vision.
(9) Feature: The feature is an intermediate-layer result after a target is input into the CNN, and is a representation in a high-dimensional space after nonlinear transformation is performed on the input.
(10) Embedding processing: The embedding process is a processing process for converting an analog signal into a corresponding digital sequence (vector) through analog-to-digital (A/D) conversion.

The following describes a method for training an electroencephalogram signal analysis model provided in the embodiments of the present disclosure based on a machine learning technology and a brain-computer interface technology.

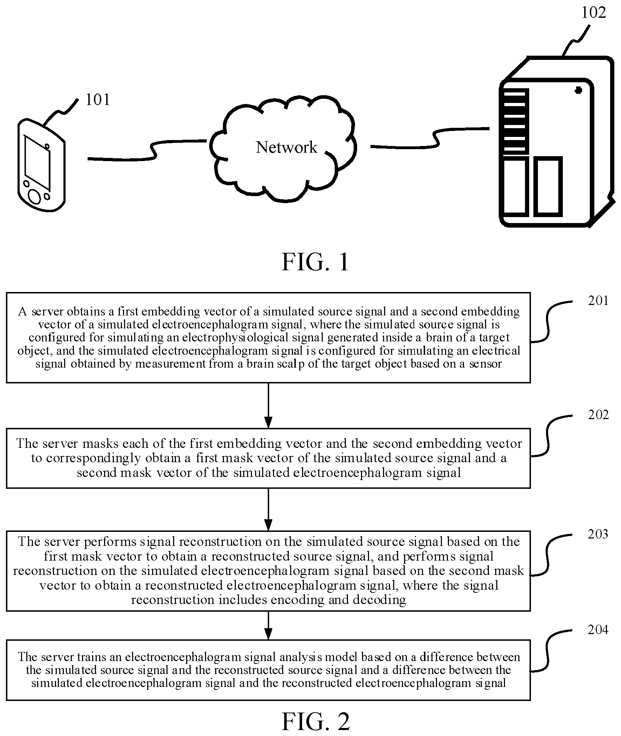

The method for training an electroencephalogram signal analysis model according to the embodiments of the present disclosure can be performed by a computer device. In some embodiments, the computer device is a terminal or a server. An implementation environment of a method for training an electroencephalogram signal analysis model according to an embodiment of the present disclosure is described below by taking an example in which a computer device is a server. FIG. 1 is a schematic diagram of an implementation environment of a method for training an electroencephalogram signal analysis model according to an embodiment of the present disclosure. Referring to FIG. 1, the implementation environment includes a terminal 101 and a server 102. The terminal 101 and the server 102 may be directly or indirectly connected in a wired or wireless communication mode. This is not limited in the present disclosure.

In some embodiments, the terminal 101 is a smartphone, a tablet computer, a notebook computer, a desktop computer, a smart speaker, a smart watch, a smart voice interaction device, a smart household appliance, a vehicle terminal, or the like, but is not limited thereto. An application having an electroencephalogram processing capability is installed on the terminal 101. The application may be a VR application, a game application, or an artificial intelligence application having an electroencephalogram processing function. This is not limited in the embodiments of the present disclosure. The application is associated with the server 102, and the server 102 provides a background service. In some embodiments, the terminal 101 may alternatively be an electronic device having a computer interface. The computer interface may obtain an electroencephalogram signal of a head of a target object through an electrode.

In some embodiments, the server 102 is an independent physical server, or can be a server cluster or distributed system including a plurality of physical servers, or can be a cloud server providing basic cloud computing services such as a cloud service, a cloud database, cloud computing, a cloud function, cloud storage, a network service, cloud communication, a middleware service, a domain name service, a security service, a content delivery network (CDN), big data, and an artificial intelligence platform. In some embodiments, the server 102 may have a data transmission interface. The data transmission interface is configured to receive an electroencephalogram signal collected by the terminal 101 having the brain-computer interface. In some embodiments, the server 102 is responsible for primary computing work, and the terminal 101 is responsible for secondary computing work. Alternatively, the server 102 is responsible for secondary computing work, and the terminal 101 is responsible for primary computing work. Alternatively, the server 102 and the terminal 101 use a distributed computing architecture for collaborative computing.

In a possible implementation of the embodiments of the present disclosure, the server 102 can perform signal reconstruction on an electroencephalogram signal to obtain a reconstructed signal based on an electroencephalogram signal analysis model, and then train an electroencephalogram signal analysis model based on a difference between the electroencephalogram signal and the reconstructed signal. After a training process of the electroencephalogram signal analysis model is completed, the server 102 transmits the trained electroencephalogram signal analysis model to the terminal 101 through a wired or wireless connection. The terminal 101 receives the trained electroencephalogram signal analysis model, and inputs data information corresponding to the electroencephalogram signal analysis model to an application having an electroencephalogram signal processing function, so that a user may process the electroencephalogram signal according to the trained electroencephalogram signal analysis model when processing the electroencephalogram signal by using the application.

FIG. 2 is a flowchart of a method for training an electroencephalogram signal analysis model according to an embodiment of the present disclosure, as shown in FIG. 2, taking an example in which a server performs the method for description in this embodiment of the present disclosure. The method for training an electroencephalogram signal analysis model includes the following operations.

201: A server obtains a first embedding vector of a simulated source signal and a second embedding vector of a simulated electroencephalogram signal, where the simulated source signal is configured for simulating an electrophysiological signal generated inside a brain of a target object, and the simulated electroencephalogram signal is configured for simulating an electrical signal measured from a brain scalp of the target object based on a sensor.

In this embodiment of the present disclosure, the simulated source signal is an electrical signal generated by simulating a real source signal. The source signal is the electrophysiological signal generated inside the brain of the target object, and is configured for reflecting a brain activity and brain connectivity information of the target object. The simulated electroencephalogram signal is an electrical signal generated by simulating a real electroencephalogram signal. The electroencephalogram signal is an electrical signal measured from the brain scalp of the target object through the sensor, and is configured for reflecting a voltage potential generated by a current within and around a neuron of the brain of the target object. The server performs embedding on each of the simulated source signal and the simulated electroencephalogram signal based on the electroencephalogram signal analysis model, so that the simulated source signal and the simulated electroencephalogram signal can be converted into vector representations, that is, an embedding vector of the simulated source signal and an embedding vector of the simulated electroencephalogram signal are obtained.

202: The server masks each of the first embedding vector and the second embedding vector to correspondingly obtain a first mask vector of the simulated source signal and a second mask vector of the simulated electroencephalogram signal.

In this embodiment of the present disclosure, the server can cover some elements in the first embedding vector by masking the first embedding vector, so as to obtain a masked first embedding vector, that is, a first mask vector, thereby increasing diversity and randomness of input data. Similarly, the server can cover some elements in the second embedding vector by masking the second embedding vector, so as to obtain a masked second embedding vector, that is, a second mask vector.

203: The server performs signal reconstruction on the simulated source signal based on the first mask vector to obtain a reconstructed source signal, and performs signal reconstruction on the simulated electroencephalogram signal based on the second mask vector to obtain a reconstructed electroencephalogram signal, where the signal reconstruction includes encoding and decoding.

In this embodiment of the present disclosure, the server can restore the first embedding vector of the simulated source signal by predicting a value of a covered element in the first embedding vector based on the electroencephalogram signal analysis model, and then signal reconstruction is performed on the simulated source signal based on the restored first embedding vector, so as to obtain the reconstructed source signal. Similarly, the second embedding vector of the simulated electroencephalogram signal can be stored by predicting a value of a covered element in the second embedding vector of the simulated electroencephalogram signal, and then signal reconstruction is performed on the simulated electroencephalogram signal based on the restored second embedding vector, so as to obtain the reconstructed electroencephalogram signal.

204: The server trains an electroencephalogram signal analysis model based on a difference between the simulated source signal and the reconstructed source signal and a difference between the simulated electroencephalogram signal and the reconstructed electroencephalogram signal.

In this embodiment of the present disclosure, the server can obtain a training loss of the electroencephalogram signal analysis model by determining the difference between the simulated source signal and the reconstructed source signal and the difference between the simulated electroencephalogram signal and the reconstructed electroencephalogram signal. The training loss is configured for reflecting training accuracy of the electroencephalogram signal analysis model, and the training loss is negatively correlated to the training accuracy. As the training loss of the electroencephalogram signal analysis model decreases, the training accuracy of the electroencephalogram signal analysis model improves, that is, the electroencephalogram signal analysis model has an enhanced decoding capability for the simulated source signal and the simulated electroencephalogram signal.

The embodiments of the present disclosure provide a method for training an electroencephalogram signal analysis model. A first embedding vector of a simulated source signal and a first embedding vector of a simulated electroencephalogram signal are respectively masked, so that a masked first embedding vector (that is, a first mask vector) and a masked second embedding vector (that is, a second mask vector) can be obtained, thereby increasing randomness of input data. Then, the first mask vector and the second mask vector are respectively encoded and decoded, so that a reconstructed source signal corresponding to the simulated source signal and a reconstructed electroencephalogram signal corresponding to the simulated electroencephalogram signal can be reconstructed. Then, the electroencephalogram signal analysis model is trained based on a difference between the simulated source signal and the reconstructed source signal and a difference between the simulated electroencephalogram signal and the reconstructed electroencephalogram signal, so that a trained electroencephalogram signal analysis model can have a universal decoding capability for electroencephalogram signals, then can accurately decode electroencephalogram information in a real electroencephalogram signal, and can be widely applied to various downstream task scenarios.

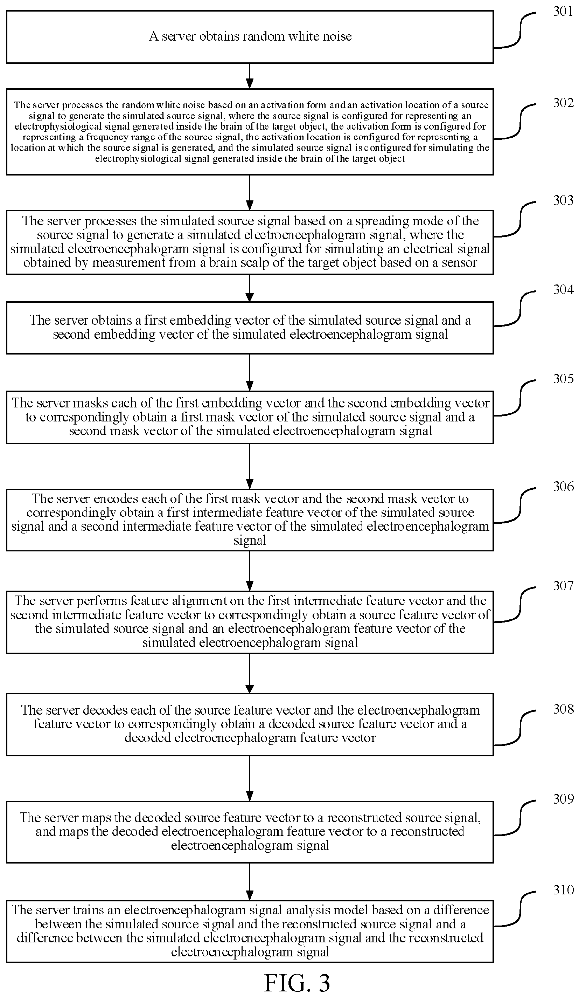

FIG. 3 is a flowchart of another method for training an electroencephalogram signal analysis model according to an embodiment of the present disclosure. As shown in FIG. 3, an example in which a server performs the method is taken for description in this embodiment of the present disclosure. The method for training an electroencephalogram signal analysis model includes the following operations.

301: A server obtains random white noise.

In this embodiment of the present disclosure, the random white noise refers to noise with power spectral density that is evenly distributed in a frequency domain. The server can generate a stable simulated source signal by processing the obtained white noise.

302: The server processes the random white noise based on an activation form and an activation location of a source signal to generate the simulated source signal, where the source signal is configured for representing an electrophysiological signal generated inside the brain of the target object, the activation form is configured for representing a frequency range of the source signal, the activation location is configured for representing a location at which the source signal is generated, and the simulated source signal is configured for simulating the electrophysiological signal generated inside the brain of the target object.

In this embodiment of the present disclosure, the source signal is the electrophysiological signal generated inside the brain of the target object, and is configured for reflecting a brain activity and brain connectivity information of the target object. The target object may be a person or an animal. The simulated source signal is an electrical signal generated by simulating a real source signal. Because there are a plurality of encephalic regions inside the brain, frequency ranges of source signals generated by different encephalic regions are different. The server can determine the frequency range of the source signal and the location at which the source signal is generated based on the activation form and the activation location of the source signal, and then process the random white noise according to the frequency range, so that the simulated source signal in a particular frequency band can be obtained, and the frequency range of the simulated source signal is consistent with that of the source signal.

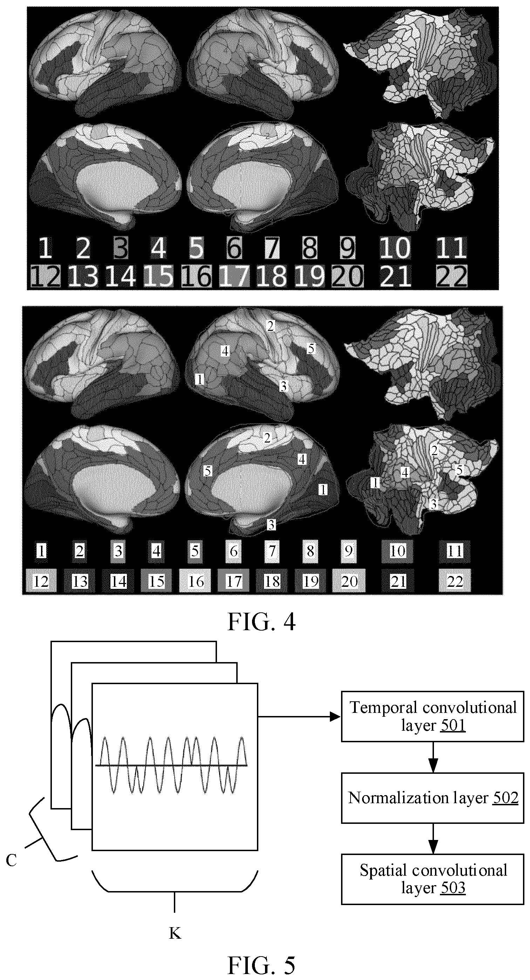

In some embodiments, for the activation location of the source signal, in the present disclosure, the brain of the target object is divided based on an encephalic region definition specification in human connectome project multi-modal parcellation version 1.0 (HCPMMP1.0). Specifically, referring to FIG. 4, in the present disclosure, 44 encephalic regions symmetrically distributed left and right are defined according to functional structural characteristics of the brain. A central point of each encephalic region is taken as the activation location of the source signal. Each activation location randomly generates a source signal with a probability of 0.5.

In some embodiments, the server performs band-pass filtering on the random white noise based on the activation form of the source signal to generate the simulated source signal in the particular frequency band. Referring to Table 1, in this embodiment of the present disclosure, five frequency bands are set in total, including main frequency ranges of electroencephalogram signals. Each source signal randomly selects one frequency band from the five frequency bands as a frequency range of the source signal with a probability of 0.2, and then band-pass filtering is performed on random white noise to generate a simulated source signal. Because different types of brain waves reflect different human states, different frequency ranges of the electroencephalograms usually express different meanings, as shown in Table 2.

**Table 1**

| Type | Frequency band |
|---|---|
| theta | 4-8 Hz |
| alpha | 10-14 Hz |
| beta_l | 16-20 Hz |
| beta_h | 22-26 Hz |
| gamma | 28-32 Hz |

**Table 2**

| Brain wave type | Frequency | Human body states |
|---|---|---|
| delta | 0.1-3 Hz | Deep sleep without dreaming |
| theta | 4-7 Hz | Adult emotion under stress |
| alpha | 8-12 Hz | Relaxed, calm, and eyes closed but awake |
| beta_l | 12.5-16 Hz | Relaxed but spiritually concentrated |
| beta_m | 16.5-20 Hz | Thinking about and processing information received from outside |
| beta_h | 20.5-28 Hz | Excited and anxious |
| gamma | 25-100 Hz | Awareness improvement, stress reduction, and meditation |
| lambda | Evoked potential | Evoked after 100 milliseconds of light stimulation to eyes |
| P300 | Evoked potential | Evoked after 300 milliseconds when seeing or listening to what a user imagines in the brain |

In some embodiments, in this solution, 44 encephalic regions can be divided and merged into 10 sets according to locations and functions. Source signals in a plurality of frequency bands at a plurality of activation locations are considered in each set, which can facilitate a subsequent task of classifying electroencephalogram signals by a model. Referring to Table 3, because the encephalic regions are left and right symmetrical, for 22 encephalic regions on any side, the 22 encephalic regions can be divided into 5 sets.

**Table 3**

| Set | Encephalic region |
|---|---|
| Set 1 | Primary visual cortex |
| | Earlier visual cortex |
| | Dorsal stream visual cortex |
| | Ventral stream visual cortex |
| | Complex and nearby regions |
| Set 2 | Somatosensory and motor cortex |
| | Paracentral lobule and cingulate central cortex |
| | Premotor cortex |
| Set 3 | Post operator cortex |
| | Earlier auditory cortex |
| | Auditory association cortex |
| | Insular and frontal elliptic cortex |
| | Medial temporal cortex |
| | Lateral temporal cortex |
| Set 4 | Temporal occipital cortex |
| | Parietal cortex |
| | Inferior parietal cortex |
| | Posterior cingulate cortex |
| Set 5 | Cingulum gyrus medial prefrontal cortex |
| | Orbit and polar frontal cortex |
| | Subfrontal cortex |
| | Dorsal lateral prefrontal cortex |

303: The server processes the simulated source signal based on a spreading mode of the source signal to generate a simulated electroencephalogram signal, where the simulated electroencephalogram signal is configured for simulating an electrical signal measured from a brain scalp of the target object based on a sensor.

In this embodiment of the present disclosure, the source signal is a signal generated inside the brain and cannot be directly obtained by measurement. To simulate and obtain the electroencephalogram signal measured from the brain scalp, the spreading mode of the source signal usually needs to be determined, that is, a mode in which the source signal diffuses from an interior of the brain to the brain scalp finally through gray matter, a skull, soft tissue, and the like of the brain in sequence. The server processes a simulated signal of the source signal, that is, the simulated source signal, based on the spreading mode of the source signal to generate a simulated electroencephalogram signal. The simulated electroencephalogram signal is an electrical signal generated by simulating a real electroencephalogram signal. The electroencephalogram signal is an electrical signal measured from the brain scalp of the target object through the sensor, and is configured for reflecting a voltage potential generated by a current within and around a neuron of the brain of the target object.

In some embodiments, the server determines the spreading mode of the source signal based on parameters of a head forward model, and then process the simulated source signal based on the spreading mode, so as to obtain the simulated electroencephalogram signal. For example, the processing performed on the simulated source signal may including filtering processing (e.g., by using a bandpass filter to remove high-frequency noise and low-frequency drift), de-artifact (e.g., by using independent component analysis to removing artifacts), or the like. The parameters of the head forward model are shown in Table 4.

**Table 4**

| Parameters | | | |
|---|---|---|---|
| Model | | Model type | Sparse model, extended model |
| | | Source signal channel | 1 to 44 |
| | | Source signal | Independent |
| | | Range | [2, 10] |
| | | Amplitude | 10 to 60 |
| | | Amplitude weight | Gaussian type |
| | | Spreading mode | Region growth |
| | | Activation form | Any |
| | | Activation location | Center |
| Simulation | Sensor noise | beta | 1 to 10 |
| | EOG noise | Amplitude | 50 to 110 |
| | Source noise | alpha | 25 to 150 |
| | | Pink noise | 25 to 120 |
| | | Sensor | 0.05 to 1.2 |

304: The server obtains a first embedding vector of the simulated source signal and a second embedding vector of the simulated electroencephalogram signal.

In some embodiments, the server performs temporal convolution on each of the simulated source signal and the simulated electroencephalogram signal to obtain a time source vector of the simulated source signal and a time electroencephalogram signal of the simulated electroencephalogram signal, then performs normalization on each of the time source vector and the time electroencephalogram signal to correspondingly obtain a normalized time source vector and a normalized time electroencephalogram signal, and then performs spatial convolution on each of the normalized time source vector and the normalized time electroencephalogram signal to obtain the first embedding vector of the simulated source signal and the second embedding vector of the simulated electroencephalogram signal.

In this embodiment of the present disclosure, the electroencephalogram signal analysis model includes an embedding module, and the embedding module includes a temporal convolutional layer, a normalization layer, and a spatial convolutional layer. The temporal convolutional layer is configured for performing feature aggregation on the simulated source signal and the simulated electroencephalogram signal in a temporal dimension. The normalization layer is configured for performing normalization on the simulated source signal and the simulated electroencephalogram signal. The spatial convolutional layer is configured for performing feature aggregation on the simulated source signal and the simulated electroencephalogram signal in a spatial dimension. The server performs, based on the temporal convolutional layer, temporal convolution on each of the simulated source signal and the simulated electroencephalogram signal, so that the time source vector of the simulated source signal and the time electroencephalogram vector of the simulated electroencephalogram signal can be obtained. Then, normalization is performed, based on the normalization layer, on each of the time source vector and the time electroencephalogram vector to correspondingly obtain a normalized time source vector and a normalized time electroencephalogram vector. Finally, spatial convolution is performed, based on the spatial convolutional layer, on each of the normalized time source vector and the time electroencephalogram signal vector to obtain the first embedding vector of the simulated source signal and the second embedding vector of the simulated electroencephalogram signal.

For example, FIG. 5 is a schematic structural diagram of an embedding module according to an embodiment of the present disclosure. The embedding module is configured to perform embedding processing on each of the simulated source signal and the simulated electroencephalogram signal, so as to convert the simulated source signal and the simulated electroencephalogram signal into vector representations. As shown in FIG. 5, C represents a quantity of channels, and K represents a size of a convolution kernel. The embedding module includes a temporal convolutional layer 501, a normalization layer 502, and a spatial convolutional layer 503. In the temporal convolutional layer, a size of a convolution kernel is 2×2, and a quantity of channels is 1, and a quantity of convolution kernels is 65. In the spatial convolutional layer, a size of a convolution kernel is 2×2, C represents a quantity of electrodes, that is, a quantity of channels for the simulated electroencephalogram signal, and is usually set as 64, and a quantity of convolution kernels is 5.

305: The server masks each of the first embedding vector and the second embedding vector to correspondingly obtain a first mask vector of the simulated source signal and a second mask vector of the simulated electroencephalogram signal.

In this embodiment of the present disclosure, the server can cover some elements in the first embedding vector by masking the first embedding vector, so as to obtain the masked first embedding vector, that is, the first mask vector. Similarly, the server can cover some elements in the second embedding vector by masking the second embedding vector, so as to obtain a masked second embedding vector, that is, a second mask vector. In this way, diversity and randomness of input data are increased.

In some embodiments, the server masks each of the first embedding vector and the second embedding vector based on a preset mask rate. The mask rate may be 50%, 60%, 70%, or the like. Taking an example in which the mask rate is 50%, half of elements in the embedding vector can be covered by masking the first embedding vector. A magnitude of the mask rate is not limited in the embodiments of the present disclosure.

306: The server encodes each of the first mask vector and the second mask vector to correspondingly obtain a first intermediate feature vector of the simulated source signal and a second intermediate feature vector of the simulated electroencephalogram signal.

In this embodiment of the present disclosure, the electroencephalogram signal analysis model further includes an encoder corresponding to the simulated source signal and an encoder corresponding to the simulated electroencephalogram signal. The encoders are configured to perform feature extraction from the simulated source signal and the simulated electroencephalogram signal. The server can encode, based on the encoders, each of the mask vector of the simulated source signal and the mask vector of the simulated electroencephalogram signal that are obtained after masking, to obtain the first intermediate feature vector of the simulated source signal and the second intermediate feature vector of the simulated electroencephalogram signal, so that the simulated source signal and the simulated electroencephalogram signal can be mapped to a common feature space, thereby facilitating joint processing on feature vectors of two types of signals.

307: The server performs feature alignment on the first intermediate feature vector and the second intermediate feature vector to correspondingly obtain a source feature vector of the simulated source signal and an electroencephalogram feature vector of the simulated electroencephalogram signal.

In this embodiment of the present disclosure, the electroencephalogram signal analysis model further includes a joint encoder. The joint encoder is configured to align features of the simulated source signal and the simulated electroencephalogram signal. The server can perform, based on the joint encoder, feature alignment on the intermediate feature vector of the simulated source signal and the intermediate feature vector of the simulated electroencephalogram signal that are obtained after encoding, to obtain the source feature vector of the simulated source signal and the electroencephalogram feature vector of the simulated electroencephalogram signal.

308: The server decodes each of the source feature vector and the electroencephalogram feature vector to correspondingly obtain a decoded source feature vector and a decoded electroencephalogram feature vector.

In this embodiment of the present disclosure, the electroencephalogram signal analysis model further includes a decoder corresponding to the simulated source signal and a decoder corresponding to the simulated electroencephalogram signal. The decoders are configured to predict covered elements in the embedding vectors, so as to restore the embedding vectors. The server can decode, based on the decoder, each of the source feature vector and the electroencephalogram feature vector that are obtained after the feature alignment, to obtain a restored first embedding vector of the simulated source signal and a restored second embedding vector of the simulated electroencephalogram signal.

In some embodiments, before encoding and decoding the simulated source signal and the simulated electroencephalogram signal, the server covers some elements of embedding vectors of the two types of signals. To restore the embedding vectors, the server can fill the feature vectors of the signals. Correspondingly, the server fills the source feature vector based on a mask mark of the simulated source signal to obtain a filled source feature vector. The mask mark of the simulated source signal is configured for representing a covered element in the embedding vector of the simulated source signal. The server fills the electroencephalogram feature vector based on a mask mark of the simulated electroencephalogram signal to obtain a filled electroencephalogram feature vector. The mask mark of the simulated electroencephalogram signal is configured for representing a covered element in the embedding vector of the simulated electroencephalogram signal. The server decodes each of the filled source feature vector and the filled electroencephalogram feature vector to correspondingly obtain the decoded source feature vector and the decoded electroencephalogram feature vector. The server can cover some elements in the embedding vectors by masking each of the first embedding vector and the second embedding vector, so as to obtain mask vectors. The server can determine a mask mark of a simulated signals based on a difference between the mask vector and the embedding vector. The mask mark is configured for representing a covered element in the embedding vector. The server can predict a value of the covered element in the embedding vector by decoding the source feature vector and the electroencephalogram feature vector. Therefore, before decoding, the server can determine a covered element in the embedding vector based on the mask mark, and then fill each of the source feature vector and the electroencephalogram feature vector by using a preset weight, so that each of the filled source feature vector and the filled electroencephalogram feature vector can be decoded to obtain a restored first embedding vector of the simulated source signal and a restored second embedding vector of the simulated electroencephalogram signal.

309: The server maps the decoded source feature vector to obtain a reconstructed source signal, and maps the decoded electroencephalogram feature vector to obtain a reconstructed electroencephalogram signal.

In this embodiment of the present disclosure, the electroencephalogram signal analysis model further includes a deconvolution module corresponding to the simulated source signal and a deconvolution module corresponding to the simulated electroencephalogram signal. The deconvolution modules are configured to perform feature mapping on the simulated source signal and the simulated electroencephalogram signal. The server can perform mapping on, based on the deconvolution modules, the decoded source feature vector of the simulated source signal to obtain a reconstructed source signal, and perform mapping on the decoded electroencephalogram feature vector of the simulated electroencephalogram signal to obtain a reconstructed electroencephalogram signal.

In some embodiments, the deconvolution module includes a deconvolutional layer and a convolutional layer. Correspondingly, the server performs deconvolution processing on the decoded source feature vector to obtain a deconvolution source vector of the simulated source signal. The server performs convolution processing on the deconvolution source vector to obtain a reconstructed source signal. The server performs deconvolution processing on the decoded electroencephalogram feature vector to obtain a deconvolution electroencephalogram vector of the simulated electroencephalogram signal. The server performs convolution processing on the deconvolution electroencephalogram vector to obtain a reconstructed electroencephalogram signal. The deconvolution module includes a deconvolutional layer and a convolutional layer. The deconvolutional layer is configured to perform deconvolution on the decoded source feature vector and the decoded electroencephalogram feature vector to obtain a deconvolution source vector of the simulated source signal and a deconvolution electroencephalogram vector of the simulated electroencephalogram signal. The convolutional layer is configured to further process the deconvolution source vector and the deconvolution electroencephalogram vector, so as to map the simulated source signal and the simulated electroencephalogram signal from a feature space back to an original space, thereby obtaining a reconstructed source signal and a reconstructed electroencephalogram signal.

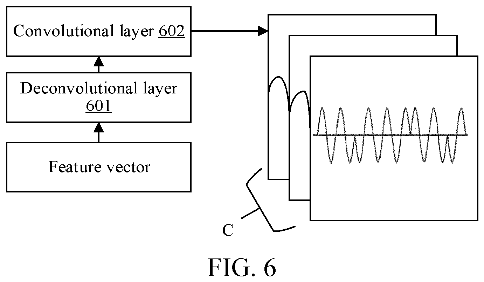

For example, FIG. 6 is a schematic structural diagram of a deconvolution module according to an embodiment of the present disclosure. The deconvolution module is configured to perform feature mapping on each of the simulated source signal and the simulated electroencephalogram signal, so as to map the source feature vector and the electroencephalogram feature vector to obtain a reconstructed source signal and a reconstructed electroencephalogram signal. As shown in FIG. 6, C represents a quantity of channels, and the deconvolution module includes a deconvolutional layer 601 and a convolutional layer 602. In the deconvolutional layer, a size of a convolution kernel is 2×2, a quantity of channels is 64, and a quantity of convolution kernels is 5. In the convolutional layer, a size of a convolution kernel is 1×1, a quantity of channels is 1, and a quantity of convolution kernels is 65.

310: The server trains an electroencephalogram signal analysis model based on a difference between the simulated source signal and the reconstructed source signal and a difference between the simulated electroencephalogram signal and the reconstructed electroencephalogram signal.

In this embodiment of the present disclosure, the server can obtain a training loss of the electroencephalogram signal analysis model by determining the difference between the simulated source signal and the reconstructed source signal and the difference between the simulated electroencephalogram signal and the reconstructed electroencephalogram signal. Then, a model parameter of the electroencephalogram signal analysis model can be updated based on the training loss, where the training loss is configured for reflecting training accuracy of the electroencephalogram signal analysis model, and the training loss is negatively correlated to the training accuracy. As the training loss of the electroencephalogram signal analysis model decreases, the training accuracy of the electroencephalogram signal analysis model improves, that is, the electroencephalogram signal analysis model has an enhanced decoding capability for the simulated source signal and the simulated electroencephalogram signal.

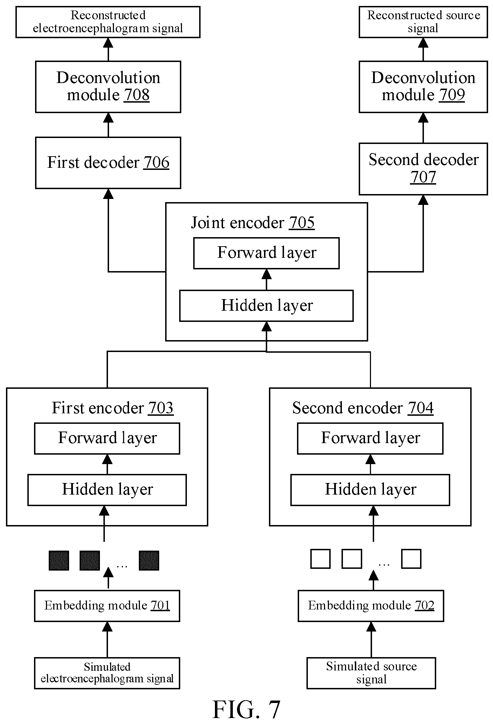

For example, FIG. 7 is a schematic structural diagram of an electroencephalogram signal analysis model according to an embodiment of the present disclosure. The electroencephalogram signal analysis model includes an embedding module 701, an embedding module 702, a first encoder 703, a second encoder 704, a joint encoder 705, a first decoder 706, a second decoder 707, a deconvolution module 708, and a deconvolution module 709. The first encoder 703, the second encoder 704, and the joint encoder 705 include a hidden layer and a forward layer. Input of the electroencephalogram signal analysis model is a simulated source signal and a simulated electroencephalogram signal. First, the simulated electroencephalogram signal and the simulated source signal are converted into embedding vectors by using the embedding modules. After being processed through the embedding modules, fixed locating code is added to the embedding vectors, and location information is added to the embedding vectors. Then, mask vectors obtained by masking the embedding vectors are respectively input to the first encoder and the second encoder to obtain an intermediate feature vector of the simulated source signal and an intermediate feature vector of the simulated electroencephalogram signal. That is, signals in two modes are mapped to a common feature space. Then, the joint encoder further performs feature alignment on the intermediate feature vectors of the two types of signals to obtain a source feature vector and an electroencephalogram feature vector. The electroencephalogram feature vector and the source feature vector are respectively input to the first decoder and the second decoder for decoding. After decoding is completed, the deconvolution module maps the electroencephalogram feature vector and the source feature vector back to original space to obtain a reconstructed electroencephalogram signal and a reconstructed source signal.

In some embodiments, specific parameters of each module in the electroencephalogram signal analysis model are shown in Table 5. The first encoder, the second encoder, the joint encoder, the first decoder, and the second encoder in the electroencephalogram signal analysis model all use a structure of Transformer. Quantities of Transformer layers are respectively 3, 3, 2, 1, 1, a hidden layer feature dimension is 64, a forward layer feature dimension is 256, an activation function is GeLU, a normalization function is BatchNorm, and Dropout= 0.1.

**Table 5**

| | First encoder | Second encoder | Joint encoder | First decoder | Second decoder |
|---|---|---|---|---|---|
| Hidden layer feature dimension | 64 | | | | |
| Forward layer feature dimension | 256 | | | | |
| Activation function | GeLU | | | | |
| Quantity of Transformer layers | 3 | 3 | 2 | 1 | 1 |
| Normalization function | BatchNorm | | | | |
| Dropout | 0.1 | | | | |

In some embodiments, the server determines a total loss of the model based on a reconstruction loss of the simulated source signal and a reconstruction loss of the simulated electroencephalogram signal. Correspondingly, the server obtains a reconstruction loss of the simulated source signal based on a difference between the simulated source signal and the reconstructed source signal. The server obtains, based on a difference between the simulated electroencephalogram signal and the reconstructed electroencephalogram signal, a reconstruction loss of the simulated electroencephalogram signal. The server performs weighted summation on the reconstruction loss of the simulated source signal and the reconstruction loss of the simulated electroencephalogram signal to obtain a training loss of the electroencephalogram signal analysis model. The server updates a parameter of the electroencephalogram signal analysis model based on the training loss. The server can determine the reconstruction loss of the simulated source signal by determining the difference between the simulated source signal and the reconstructed source signal, and similarly, can determine the reconstruction loss of the simulated electroencephalogram signal by determining the difference between the simulated electroencephalogram signal and the reconstructed electroencephalogram signal. Then, weighted summation is performed on the reconstruction losses corresponding to the two types of signals to determine the total loss of the model, namely, the training loss of the electroencephalogram signal analysis model. Then, based on the training loss, the model parameter of the electroencephalogram signal analysis model can be updated, so that the training loss is reduced, and the updated electroencephalogram signal analysis model is obtained through training. The model is trained by combining the simulated signals in two modes, so that the model can be promoted to align the signal features of the simulated source signal and the simulated electroencephalogram signal, thereby improving a universal decoding capability of the electroencephalogram signal analysis model to an electroencephalogram signal.

In some embodiments, the training loss of the electroencephalogram signal analysis model is determined according to the following formula (1). $L = γ \frac{{M}_{x} ⋅ {\left‖X-{X}^{˜}\right‖}_{2}}{numel \left({M}_{x}\right)} + \frac{{M}_{s} ⋅ {\left‖S-{S}^{˜}\right‖}_{2}}{numel \left({M}_{s}\right)}$
where L is the training loss of the electroencephalogram signal analysis model, *X̃*, *S̃* are respectively the reconstructed electroencephalogram signal and the reconstructed source signal; *Mₓ* and *Mₛ* are respectively a mask of the simulated electroencephalogram signal and a mask of the simulated source signal; and in the masks, 1 is a covered part, and 0 is a remaining part; Numel (•) indicates counting quantities of non-zero locations in *Mₓ* and *Mₛ,* and is configured for normalizing; γ is a hyper-parameter, and is configured for balancing the reconstruction loss of the simulated source signal and the reconstruction loss of the simulated electroencephalogram signal γ = 1.

In some embodiments, the server can construct a target electroencephalogram signal model based on a target embedding module and a first encoder in the electroencephalogram signal analysis model, and fine-tune the target electroencephalogram signal model by using a real electroencephalogram signal. Correspondingly, the server constructs a target electroencephalogram signal model based on a target embedding module, a first encoder, and a first decoder. The first decoder is configured to decode an electroencephalogram feature vector of a real electroencephalogram signal, the target electroencephalogram signal model is configured for performing signal reconstruction on an electroencephalogram signal, and the real electroencephalogram signal is configured for representing an electrical signal measured from a brain scalp of a target object based on a sensor. The server performs signal reconstruction on the real electroencephalogram signal based on the target electroencephalogram signal model to obtain a reconstructed signal. The server trains the target electroencephalogram signal model based on a difference between the real electroencephalogram signal and the reconstructed signal. The target embedding module is an embedding module for embedding the simulated electroencephalogram signal in the electroencephalogram signal analysis model. The first decoder is an encoder for encoding an embedding vector of a simulated electroencephalogram signal in the electroencephalogram signal analysis model. The server can construct the target electroencephalogram signal model based on the target embedding module, the first encoder, and the first decoder that is configured to decode the electroencephalogram feature vector of the real electroencephalogram signal. The server can embed, encode, and decode the real electroencephalogram signal based on the target embedding module, the first encoder, and the first decoder in the target electroencephalogram signal model, so as to reconstruct a reconstructed signal corresponding to the real electroencephalogram signal. Then, the target electroencephalogram signal analysis model is trained based on a difference between the real electroencephalogram signal and the reconstructed signal, so that the trained target electroencephalogram signal model can accurately decode electroencephalogram information in the real electroencephalogram signal. Based on a universal decoding capability of the model to the electroencephalogram signal, a capability of the model to decode the real electroencephalogram signal is further improved.

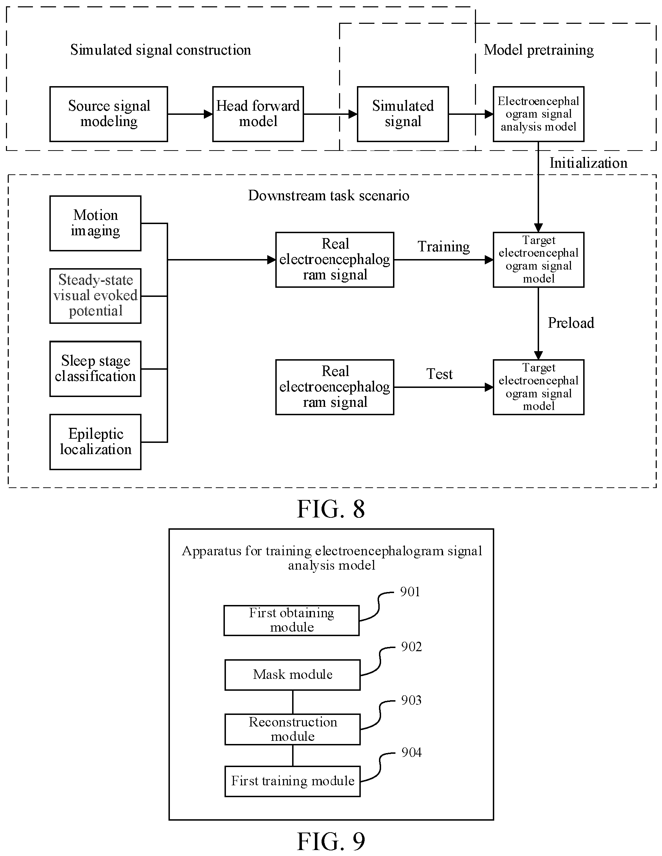

For example, FIG. 8 is a schematic diagram of a training process of an electroencephalogram signal analysis model according to an embodiment of the present disclosure. As shown in FIG. 8, first, this embodiment of the present disclosure may be divided into three parts. The first part is to construct a simulated signal. First, a source signal is modeled, that is, random noise is processed according to an activation form and an activation location of the source signal to obtain a simulated source signal. Then, a spreading mode of the source signal can be determined according to a parameter in the head forward model. The simulated source signal is processed to obtain a simulated electroencephalogram signal. The second part is to pretrain the electroencephalogram signal analysis model by using the simulated electroencephalogram signal and the simulated source signal, so as to improve a general decoding capability of the model to the electroencephalogram signal. The third part is for a plurality of downstream task scenarios such as motion imaging, steady-state visual evoked potential, sleep stage classification, and epileptic localization. In this embodiment of the present disclosure, a pretrained electroencephalogram signal analysis model can be fine-tuned by using the real electroencephalogram signal, that is, a target electroencephalogram signal model corresponding to a downstream task uses an embedding module and an encoder that are the same as those of the target electroencephalogram signal model, and a decoder of the target electroencephalogram signal model is designed according to a specific downstream task. Before the target electroencephalogram signal model is fine-tuned by using the real electroencephalogram signal, first, pretraining weights of the embedding module and the encoder are loaded, the decoder is initialized, and then fine-tuning training is performed on the target electroencephalogram signal model according to the real electroencephalogram signal provided by the downstream task. After the target electroencephalogram signal model is trained, the target electroencephalogram signal model is loaded, so that the model can process the real electroencephalogram signal, and a decoding capability of the model to the real electroencephalogram signal can be tested.

The embodiments of the present disclosure provide a method for training an electroencephalogram signal analysis model. A first embedding vector of a simulated source signal and a second embedding vector of a simulated electroencephalogram signal are respectively masked, so that a masked first embedding vector (that is, a first mask vector) and a masked second embedding vector (that is, a second mask vector) can be obtained. In this way, randomness of input data can be increased. Then, the first mask vector and the second mask vector are respectively encoded and decoded, so that a reconstructed source signal corresponding to the simulated source signal and a reconstructed electroencephalogram signal corresponding to the simulated electroencephalogram signal can be reconstructed. Then, the electroencephalogram signal analysis model is trained based on a difference between the simulated source signal and the reconstructed source signal and a difference between the simulated electroencephalogram signal and the reconstructed electroencephalogram signal, so that a trained electroencephalogram signal analysis model can have a universal decoding capability for electroencephalogram signals, then can accurately decode electroencephalogram information in a real electroencephalogram signal, and can be widely applied to various downstream task scenarios.

In the embodiments of the present disclosure, user-related data such as the real electroencephalogram signal is involved. When the embodiments of the present disclosure are applied to a specific product or technology, user permission or consent needs to be obtained, and collection, use, and processing of relevant data need to comply with relevant laws, regulations, and standards of relevant countries and regions.

FIG. 9 is a block diagram of an apparatus for training an electroencephalogram signal analysis model according to an embodiment of the present disclosure. The apparatus is configured to perform the foregoing method for training an electroencephalogram signal analysis model. Referring to FIG. 9, the apparatus includes: a first obtaining module 901, a mask module 902, a reconstruction module 903, and a training module 904.

The first obtaining module 901 is configured to obtain a first embedding vector of a simulated source signal and a second embedding vector of a simulated electroencephalogram signal, where the simulated source signal is configured for simulating an electrophysiological signal generated inside a brain of a target object, and the simulated electroencephalogram signal is configured for simulating an electrical signal measured from a brain scalp of the target object based on a sensor.

The mask module 902 is configured to mask each of the first embedding vector and the second embedding vector to correspondingly obtain a first mask vector of the simulated source signal and a second mask vector of the simulated electroencephalogram signal.

The reconstruction module 903 is configured to: perform signal reconstruction on the simulated source signal based on the first mask vector to obtain a reconstructed source signal, and perform signal reconstruction on the simulated electroencephalogram signal based on the second mask vector to obtain a reconstructed electroencephalogram signal, the signal reconstruction including encoding and decoding.

The first training module 904 is configured to train an electroencephalogram signal analysis model based on a difference between the simulated source signal and the reconstructed source signal and a difference between the simulated electroencephalogram signal and the reconstructed electroencephalogram signal.

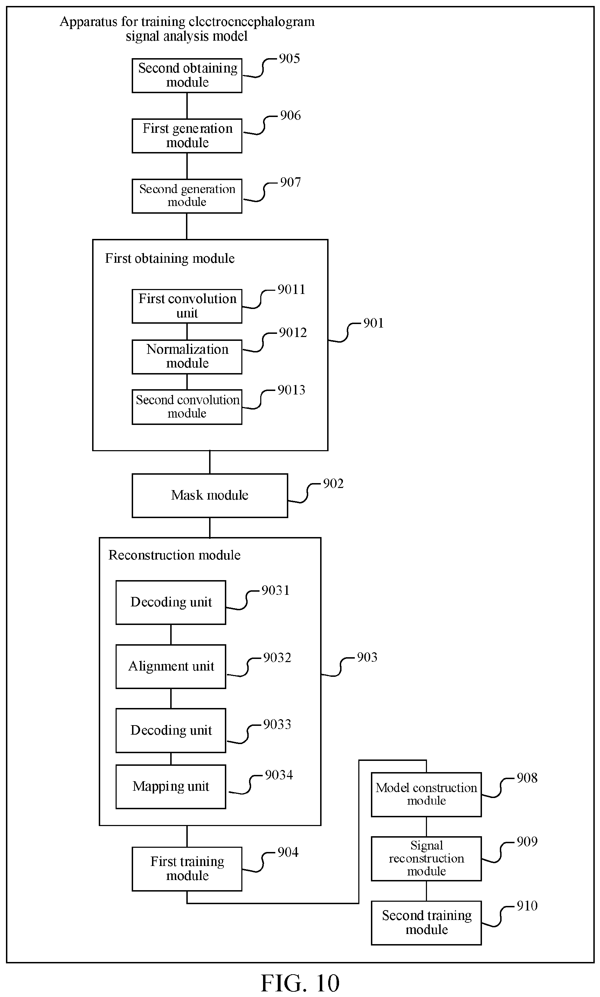

In some embodiments, FIG. 10 is a block diagram of an apparatus for training an electroencephalogram signal analysis model according to an embodiment of the present disclosure. Referring to FIG. 10, the reconstruction module 903 includes: an encoding unit 9031, configured to encode each of the first mask vector and the second mask vector to correspondingly obtain a first intermediate feature vector of the simulated source signal and a second intermediate feature vector of the simulated electroencephalogram signal; an alignment unit 9032, configured to perform feature alignment on the first intermediate feature vector and the second intermediate feature vector to obtain a source feature vector of the simulated source signal and an electroencephalogram feature vector of the simulated electroencephalogram signal; a decoding unit 9033, configured to decode each of the source feature vector and the electroencephalogram feature vector to corresponding obtain a decoded source feature vector and a decoded electroencephalogram feature vector; and a mapping unit 9034, configured to perform mapping on the decoded source feature vector to obtain the reconstructed source signal, and perform mapping on the decoded electroencephalogram feature vector to obtain the reconstructed electroencephalogram signal.

In some embodiments, the decoding unit 9033 is configured to: fill the source feature vector based on a mask mark of the simulated source signal to obtain a filled source feature vector, where the mask mark of the simulated source signal is configured for representing a covered element in an embedding vector of the simulated source signal; fill the electroencephalogram feature vector based on a mask mark of the simulated electroencephalogram signal to obtain a filled electroencephalogram feature vector, where the mask mark of the simulated electroencephalogram signal is configured for representing a covered element in an embedding vector of the simulated electroencephalogram signal; and decode each of the filled source feature vector and the filled electroencephalogram feature vector to correspondingly obtain the decoded source feature vector and the decoded electroencephalogram feature vector.

In some embodiments, the mapping unit 904 is configured to perform deconvolution processing on the decoded source feature vector to obtain a deconvolution source vector of the simulated source signal; perform convolution processing on the deconvolution source vector to obtain a reconstructed source signal; perform deconvolution processing on the decoded electroencephalogram feature vector to obtain a deconvolution electroencephalogram vector of the simulated electroencephalogram signal; and perform convolution processing on the deconvolution electroencephalogram vector to obtain a reconstructed electroencephalogram signal.

In some embodiments, continuing to refer to FIG. 10, the apparatus further includes: a second obtaining module 905, configured to obtain random white noise; a first generation module 906, configured to process the random white noise based on an activation form and an activation location of a source signal to generate the simulated source signal, where the source signal is configured for representing an electrophysiological signal generated inside a brain of a target object, the activation form is configured for representing a frequency range of the source signal, and the activation location is configured for representing a location at which the source signal is generated; and a second generation module 907, configured to process the simulated source signal based on a spreading manner of the source signal to generate the simulated electroencephalogram signal.

In some embodiments, the first training module 904 is configured to obtain a reconstruction loss of the simulated source signal based on a difference between the simulated source signal and the reconstructed source signal; obtain a reconstruction loss of the simulated electroencephalogram signal based on a difference between the simulated electroencephalogram signal and the reconstructed electroencephalogram signal; perform weighted summation on the reconstruction loss of the simulated source signal and the reconstruction loss of the simulated electroencephalogram signal to obtain a training loss of the electroencephalogram signal analysis model; and update a parameter of the electroencephalogram signal analysis model based on the training loss.

In some embodiments, continuing to refer to FIG. 10, the first obtaining module 901 includes: a first convolution unit 9011, configured to perform temporal convolution on the simulated source signal and the simulated electroencephalogram signal to obtain a time source vector of the simulated source signal and a time electroencephalogram vector of the simulated electroencephalogram signal; a normalization unit 9012, configured to normalize each of the time source vector and the time electroencephalogram vector to correspondingly obtain a normalized time source vector and a normalized time electroencephalogram vector; and a second convolution unit 9013, configured to perform spatial convolution on each of the normalized time source vector and the normalized time electroencephalogram vector to correspondingly obtain the embedding vector of the simulated source signal and the embedding vector of the simulated electroencephalogram signal.

In some embodiments, the electroencephalogram signal analysis model includes a target embedding module and a first encoder, the target embedding module is configured to perform embedding processing on the simulated electroencephalogram signal, and the first encoder is configured to encode the embedding vector of the simulated electroencephalogram signal.

Continuing to refer to FIG. 10, the apparatus further includes: a model construction module 908, configured to construct a target electroencephalogram signal model based on the target embedding module, the first encoder, and a first decoder, where the first decoder is configured to decode an electroencephalogram feature vector of a real electroencephalogram signal, the target electroencephalogram signal model is configured for performing signal reconstruction on the electroencephalogram signal, and the real electroencephalogram signal is configured for representing the electrical signal measured from the brain scalp of the target object based on the sensor; a signal reconstruction module 909, configured to perform signal reconstruction on the real electroencephalogram signal based on the target electroencephalogram signal model to obtain a reconstructed signal; and a second training module 910, configured to train the target electroencephalogram signal model based on a difference between the real electroencephalogram signal and the reconstructed signal.

The embodiments of the present disclosure provide an apparatus for training an electroencephalogram signal analysis model. An embedding vector of a simulated source signal and an embedding vector of a simulated electroencephalogram signal are respectively masked, so that a masked embedding vector of the simulated source signal and a masked embedding vector of the simulated electroencephalogram signal, that is, masked vectors, can be obtained, thereby increasing randomness of input data. Then, the mask vector of the simulated source signal and the mask vector of the simulated electroencephalogram signal are encoded and decoded, so that a reconstructed source signal corresponding to the simulated source signal and a reconstructed electroencephalogram signal corresponding to the simulated electroencephalogram signal can be reconstructed. Then, the electroencephalogram signal analysis model is trained based on a difference between the simulated source signal and the reconstructed source signal and a difference between the simulated electroencephalogram signal and the reconstructed electroencephalogram signal, so that a trained electroencephalogram signal analysis model can have a universal decoding capability for electroencephalogram signals, then can accurately decode electroencephalogram information in a real electroencephalogram signal, and can be widely applied to various downstream task scenarios.

The apparatus for training an electroencephalogram signal analysis model provided in the foregoing embodiments is only described by taking an example of division of various functional modules described above. In practical applications, the foregoing functions may be allocated to be completed by different functional modules according to requirements. That is, an internal structure of a terminal is divided into different functional modules to complete all or some of the functions described above. In addition, the apparatus for training an electroencephalogram signal analysis model belongs to a same concept as the method for training an electroencephalogram signal analysis model provided in the foregoing embodiments. For a specific implementation process of the apparatus, refer to the method embodiments.

In the embodiments of the present disclosure, the computer device may be configured as a terminal or a server. When the computer device is configured as a server, the server may be used as an execution body to implement the technical solutions provided in the embodiments of the present disclosure. When the computer device is configured as a terminal, the terminal may be used as an execution body to implement the technical solutions provided in the embodiments of the present disclosure. This is not limited in the embodiments of the present disclosure.

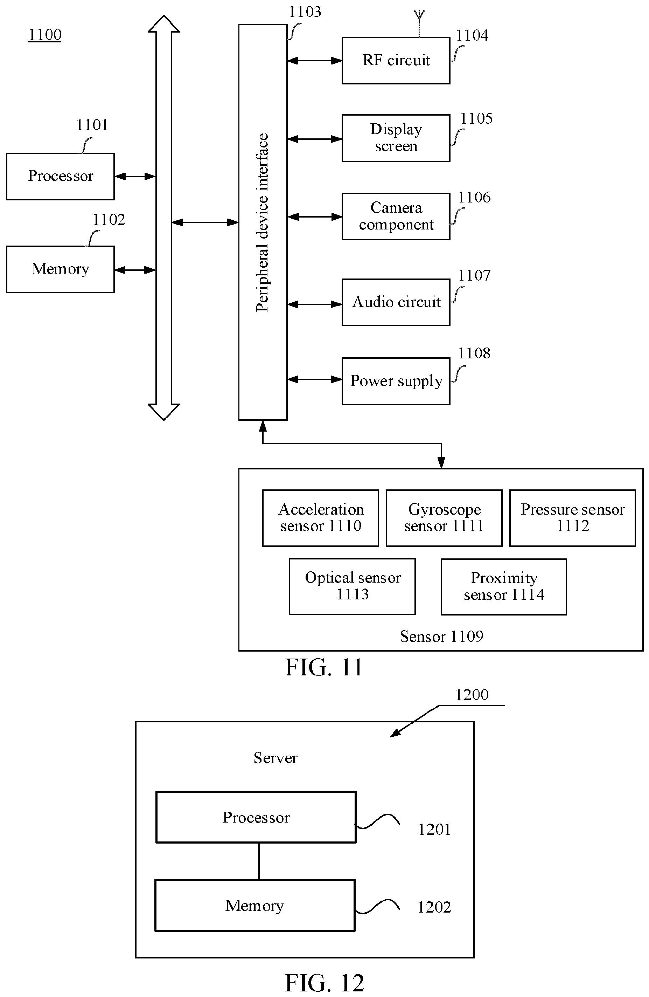

FIG. 11 is a structural block diagram of a terminal 1100 according to an embodiment of the present disclosure. The computer 1100 may be a portable mobile terminal, for example: a smartphone, a tablet computer, a moving picture experts group audio layer III (MP3) player, a moving picture experts group audio layer IV (MP4) player, a notebook computer, or a desktop computer. The terminal 1100 may alternatively be referred to as another name such as user equipment, a portable terminal, a laptop terminal, or a desktop terminal.

Generally, the terminal 1100 includes: a processor 1101 and a memory 1102.

The processor 1101 may include one or more processing cores, for example, a 4-core processor or an 8-core processor. The processor 1101 may be implemented in at least one hardware form of a digital signal processor (DSP), a field-programmable gate array (FPGA), and a programmable logic array (PLA). The processor 1101 may alternatively include a main processor and a coprocessor. The main processor is a processor configured to process data in an awake state, is alternatively referred to as a central processing unit (CPU). The coprocessor is a low-power-consumption processor configured to process data in a standby state. In some embodiments, the processor 1101 may be integrated with a graphics processing unit (GPU). The GPU is configured to render and draw content that needs to be displayed on a display screen. In some embodiments, the processor 1101 may alternatively include an artificial intelligence (AI) processor. The AI processor is configured to process computing operations related to machine learning.

The memory 1102 may include one or more computer-readable storage media. The computer-readable storage medium may be non-transient. The memory 1102 may alternatively include a high-speed random access memory and a nonvolatile memory, for example, one or more disk storage devices or flash storage devices. In some embodiments, a non-transient computer-readable storage medium in the memory 1102 is configured for storing at least one computer program. The at least one computer program is configured to be executed by the processor 1101 to implement the method for training an electroencephalogram signal analysis model provided in the embodiments of the present disclosure.

In some embodiments, the terminal 1100 may alternatively include: a peripheral device interface 1103 and at least one peripheral device. The processor 1101, the memory 1102, and the peripheral interface 1103 may be connected through a bus or a signal cable. Each peripheral device may be connected to the peripheral device interface 1103 through a bus, a signal cable, or a circuit board. Specifically, the peripheral device includes: at least one of a radio frequency (RF) circuit 1104, a display screen 1105, a camera component 1106, an audio circuit 1107, or a power supply 1108.

The peripheral interface 1103 may be configured to connect the at least one peripheral device related to an input/output (I/O) to the processor 1101 and the memory 1102. In some embodiments, the processor 1101, the memory 1102, and the peripheral device interface 1103 are integrated on the same core or circuit board. In some embodiments, any one or two of the processors 1101, the memory 1102, and the peripheral device interface 1103 may be implemented on a single chip or circuit board indecently. This is not limited in the embodiments of the present disclosure.

The RF circuit 1104 is configured to receive and transmit an RF signal, alternatively referred to as an electromagnetic signal. The RF circuit 1104 communicates with a communication network and other communication devices through the electromagnetic signal. The RF circuit 1104 converts an electrical signal into an electromagnetic signal for transmitting, or converts a received electromagnetic signal into an electrical signal. In some embodiments, the RF circuit 1104 includes: an antenna system, an RF transceiver, one or more amplifiers, a tuner, an oscillator, a digital signal processor, an encoding and decoding chip set, a subscriber identity module card, and the like. The RF circuit 1104 may communicate with another terminal by using at least one wireless communication protocol. The wireless communication protocol includes, but is not limited to: a world wide web, a metropolitan area network, an intranet, generations of mobile communication networks (2G, 3G, 4G, and 5G), a wireless local area network, and/or a wireless fidelity (Wi-Fi) network. In some embodiments, the RF 1104 may alternatively include a circuit related to NFC. This is not limited in the present disclosure.

The display screen 1105 is configured to display a user interface (UI). The UI may include a graph, text, an icon, a video, and any combination thereof. When the display screen 1105 is a touch display screen, the display screen 1105 further has a capability of collecting a touch signal on or above a surface of the display screen 1105. The touch signal may be inputted to the processor 1101 as a control signal for processing. In this case, the display screen 1105 may alternatively be configured to provide a virtual button and/or a virtual keyboard that are/is alternatively referred to as a soft button and/or a soft keyboard. In some embodiments, there may be one display screen 1105, disposed on a front panel of the terminal 1100. In some other embodiments, there may be at least two display screens 1105, respectively arranged on different surfaces of the terminal 1100 or in a folded design. In some other embodiments, the display screen 1105 may be a flexible display arranged on a curved surface or a folded surface of the terminal 1100. Even, the display screen 1105 may be further set in a non-rectangular irregular pattern, namely, a special-shaped screen. The display screen 1105 may be prepared by using materials such as a liquid crystal display (LCD), an organic light-emitting diode (OLED), or the like.

The camera component 1106 is configured to capture images or videos. In some embodiments, the camera component 1106 includes a front camera and a rear camera. Generally, the front-facing camera is disposed on the front panel of the terminal, and the rear-facing camera is disposed on a back surface of the terminal. In some embodiments, there are at least two rear cameras, which are respectively any of a main camera, a depth-of-field camera, a wide-angle camera, and a telephoto camera, to achieve background blur through fusion of the main camera and the depth-of-field camera, panoramic photographing and virtual reality (VR) photographing through fusion of the main camera and the wide-angle camera, or other fusion photographing functions. In some embodiments, the camera component 1106 may alternatively include a flash. The flash may be a monochrome temperature flash, or may be a double color temperature flash. The double color temperature flash refers to a combination of a warm light flash and a cold light flash, and may be configured for light compensation under different color temperatures.

The audio circuit 1107 may include a microphone and a speaker. The microphone is configured to collect sound waves of a user and an environment, and convert the sound waves into an electrical signal to input to the processor 1101 for processing, or input to the radio frequency circuit 1104 for implementing voice communication. For the purpose of stereo collection or noise reduction, there may be a plurality of microphones, respectively disposed at different parts of the terminal 1100. The microphone may further be an array microphone or an omni-directional acquisition type microphone. The speaker is configured to convert an electrical signal from the processor 1101 or the RF circuit 1104 into a sound wave. The speaker may be a conventional film speaker, or may be a piezoelectric ceramic speaker. When the speaker is the piezoelectric ceramic speaker, the speaker not only can convert an electrical signal into acoustic waves audible to a human being, but also can convert an electrical signal into acoustic waves inaudible to a human being, for ranging and other purposes. In some embodiments, the audio circuit 1107 may alternatively include an earphone jack.

The power supply 1108 is configured to supply power to various components in the terminal 1100. The power supply 1108 may be an alternating current, a direct current, a primary battery, or a rechargeable battery. When the power supply 1108 includes a rechargeable battery, and the rechargeable battery may be a wired rechargeable battery or a wireless rechargeable battery. The wired rechargeable battery is a battery charged through a wired circuit, and the wireless rechargeable battery is a battery charged through a wireless coil. The rechargeable battery may be further configured to support a fast charging technology.

In some embodiments, the terminal 1100 further includes one or more sensors 1109. The one or more sensors 1109 include, but are not limited to: an acceleration sensor 1110, a gyroscope sensor 1111, a pressure sensor 1112, an optical sensor 1113, and a proximity sensor 1114.

The acceleration sensor 1110 may detect a magnitude of acceleration on three coordinate axes of a coordinate system established with the terminal 1100. For example, the acceleration sensor 1110 may be configured to detect components of gravity acceleration on the three coordinate axes. The processor 1101 may control, according to a gravity acceleration signal collected by the acceleration sensor 1110, the display screen 1105 to display the UI in a landscape view or a portrait view. The acceleration sensor 1110 may be further configured to collect motion data of a game or a user.

The gyroscope sensor 1111 may detect a body direction and a rotation angle of the terminal 1100. The gyroscope sensor 1111 may cooperate with the acceleration sensor 1110 to collect a 3D action by the user on the terminal 1100. The processor 1101 may implement the following functions according to the data collected by the gyroscope sensor 1111: motion sensing (such as changing the UI according to a tilt operation of the user), image stabilization at shooting, game control, and inertial navigation.

The pressure sensor 1112 may be disposed at a side frame of the terminal 1100 and/or a lower layer of the display screen 1105. When the pressure sensor 1112 is disposed at the side frame of the terminal 1100, a holding signal of the user on the terminal 1100 may be detected. The processor 1101 performs left and right hand recognition or a quick operation based on the holding signal collected by the pressure sensor 1112. When the pressure sensor 1112 is disposed on a low layer of the display screen 1105, the processor 1101 controls, according to a pressure operation of the user on the display screen 1105, an operable control on the UI. The operable control includes at least one of a button control, a scroll-bar control, an icon control, and a menu control.

The optical sensor 1113 is configured to collect ambient light intensity. In an embodiment, the processor 1101 may control display brightness of the display screen 1105 based on the ambient light intensity collected by the optical sensor 1113. Specifically, when the ambient light intensity is relatively high, the display brightness of the display screen1105 is increased. When the ambient light intensity is relatively low, the display brightness of the display screen 1105 is decreased. In another embodiment, the processor 1101 may further dynamically adjust a camera parameter of the camera component 1106 according to the ambient light intensity collected by the optical sensor 1113.

The proximity sensor 1114, alternatively referred to as a distance sensor, is generally disposed on the front panel of the terminal 1100. The proximity sensor 1114 is configured to collect a distance between a user and a front surface of the terminal 1100. In an embodiment, when the proximity sensor 1114 detects that the distance between the user and the front surface of the computer device 1100 gradually increases, the processor 1101 controls the display screen 1105 to switch from a screen-off state to a screen-on state. When the proximity sensor 1114 detects that the distance between the user and the front surface of the terminal 1100 gradually increases, the processor 1101 controls the display screen 1105 to switch from the screen-off state to the screen-on state.

A person skilled in the art may understand that, the structure shown in FIG. 11 does not constitute a limitation on the terminal 1100, and may include more or fewer components than those shown in the figure, or combine some components, or use a different component arrangement.

FIG. 12 is a schematic structural diagram of a server according to an embodiment of the present disclosure. A server 1200 may produce a great difference due to differences in configuration or performance, and may include one or more central processing units (CPUs) 1201 and one or more memories 1202. The memory 1202 has at least one computer program stored therein. The at least one computer program is loaded and executed by the processor 1201 to implement the method for training an electroencephalogram signal analysis model provided in various method embodiments described above. Certainly, the server may further have components such as a wired or wireless network interface, a keyboard, and an I/O interface to facilitate inputting and outputting. The server may alternatively include another component for implementing a device function.

The embodiments of the present disclosure further provide a computer-readable storage medium. The computer-readable storage medium stores at least one computer program. The at least one computer program is loaded and executed by a processor to implement the method for training an electroencephalogram signal analysis model the foregoing embodiments. For example, the computer-readable storage medium may be a read-only memory (ROM), a random access memory (RAM), a compact disc read-only memory (CD-ROM), a magnetic tape, a floppy disk, an optical data storage device, or the like.

The embodiments of the present disclosure further provide a computer program product, including a computer program. The computer program implements, when executed by a processor, the method for training an electroencephalogram signal analysis model in the foregoing embodiments.

A person of ordinary skill in the art may understand that all or some of the operations of the foregoing embodiments may be implemented by hardware, or may be implemented by a program instructing relevant hardware. The program may be stored in a computer-readable storage medium. The storage medium may be a read-only memory, a magnetic disk, an optical disc, or the like.

The above descriptions are merely optional embodiments of the application, but are not intended to limit the application. Any modification, equivalent replacement, or improvement made without departing from the principle of the application shall fall within the scope of the protection of the application.

## Claims

1. A method for training an electroencephalogram signal analysis model, performed by a server, the method comprising:
obtaining (301) random white noise;
processing (302) the random white noise based on an activation form and an activation location of a source signal to generate a simulated source signal, the source signal representing an electrophysiological signal generated inside the brain of the target object, the activation form representing a frequency range of the source signal, and the activation location representing a location at which the source signal is generated;
processing (303) the simulated source signal based on a spreading mode of the source signal to generate a simulated electroencephalogram signal;
obtaining (201, 304) a first embedding vector of the simulated source signal and a second embedding vector of the simulated electroencephalogram signal based on an electroencephalogram signal analysis model, the simulated source signal indicating an electrophysiological signal generated inside a brain of a target object, and the simulated electroencephalogram signal indicating an electrical signal measured from a brain scalp of the target object based on a sensor;
masking (202, 305) the first embedding vector and the second embedding vector respectively to obtain a first mask vector of the simulated source signal and a second mask vector of the simulated electroencephalogram signal, wherein the first mask vector is a vector obtained by covering part of elements in the first embedding vector, and the second mask vector is a vector obtained by covering part of elements in the second embedding vector;
performing (203) signal reconstruction on the simulated source signal based on the first mask vector to obtain a reconstructed source signal, and performing signal reconstruction on the simulated electroencephalogram signal based on the second mask vector to obtain a reconstructed electroencephalogram signal, the signal reconstruction comprising encoding and decoding, **characterized in that** the performing (203) comprises:
encoding (306) the first mask vector and the second mask vector respectively to obtain a first intermediate feature vector of the simulated source signal and a second intermediate feature vector of the simulated electroencephalogram signal;
performing (307) feature alignment on the first intermediate feature vector and the second intermediate feature vector to obtain a source feature vector of the simulated source signal and an electroencephalogram feature vector of the simulated electroencephalogram signal;
filling the source feature vector based on a mask mark of the simulated source signal to obtain a filled source feature vector, the mask mark of the simulated source signal representing a covered element in the embedding vector of the simulated source signal;
filling the electroencephalogram feature vector based on a mask mark of the simulated electroencephalogram signal to obtain a filled electroencephalogram feature vector, the mask mark of the simulated electroencephalogram signal representing a covered element in the embedding vector of the simulated electroencephalogram signal; and
decoding the filled source feature vector and the filled electroencephalogram feature vector respectively to obtain the decoded source feature vector and the decoded electroencephalogram feature vector; and
performing mapping (309) on the decoded source feature vector to obtain the reconstructed source signal, and performing mapping on the decoded electroencephalogram feature vector to obtain the reconstructed electroencephalogram signal; and
obtaining a training loss of the electroencephalogram signal analysis model based on a difference between the simulated source signal and the reconstructed source signal and a difference between the simulated electroencephalogram signal and the reconstructed electroencephalogram signal, and updating a parameter of the electroencephalogram signal analysis model based on the training loss.

2. The method according to claim 1, wherein the performing mapping (309) on the decoded source feature vector to obtain the reconstructed source signal, and performing mapping on the decoded electroencephalogram feature vector to obtain the reconstructed electroencephalogram signal comprises:
performing deconvolution processing on the decoded source feature vector to obtain a deconvolution source vector of the simulated source signal;
performing convolution processing on the deconvolution source vector to obtain the reconstructed source signal;
performing deconvolution processing on the decoded electroencephalogram feature vector to obtain a deconvolution electroencephalogram vector of the simulated electroencephalogram signal; and
performing convolution processing on the deconvolution electroencephalogram vector to obtain the reconstructed electroencephalogram signal.

3. The method according to any one of claims 1 to 2, wherein the obtaining (201, 304) a first embedding vector of a simulated source signal and a second embedding vector of a simulated electroencephalogram signal comprises:
performing temporal convolution on the simulated source signal and the simulated electroencephalogram signal respectively to obtain a time source vector of the simulated source signal and a time electroencephalogram vector of the simulated electroencephalogram signal;
performing normalization on the time source vector and the time electroencephalogram vector respectively to obtain a normalized time source vector and a normalized time electroencephalogram vector; and
performing spatial convolution on the normalized time source vector and the normalized time electroencephalogram vector respectively to obtain the first embedding vector of the simulated source signal and the second embedding vector of the simulated electroencephalogram signal.

4. The method according to any one of claims 1 to 2, wherein the obtain a training loss of the electroencephalogram signal analysis model based on a difference between the simulated source signal and the reconstructed source signal and a difference between the simulated electroencephalogram signal and the reconstructed electroencephalogram signal comprises:
obtaining a reconstruction loss of the simulated source signal based on the difference between the simulated source signal and the reconstructed source signal;
obtaining a reconstruction loss of the simulated electroencephalogram signal based on the difference between the simulated electroencephalogram signal and the reconstructed electroencephalogram signal; and
performing weighted summation on the reconstruction loss of the simulated source signal and the reconstruction loss of the simulated electroencephalogram signal to obtain the training loss of the electroencephalogram signal analysis model.

5. The method according to any one of claims 1 to 2, wherein the electroencephalogram signal analysis model comprises a target embedding module and a first encoder, the target embedding module is configured to perform embedding processing on the simulated electroencephalogram signal, and the first encoder is configured to encode the embedding vector of the simulated electroencephalogram signal; and
the method further comprises:
constructing a target electroencephalogram signal model based on the target embedding module, the first encoder, and a first decoder, the first decoder being configured to decode an electroencephalogram feature vector of a real electroencephalogram signal, the target electroencephalogram signal model performing signal reconstruction on the electroencephalogram signal, and the real electroencephalogram signal representing the electrical signal measured from the brain scalp of the target object based on the sensor;
performing signal reconstruction on the real electroencephalogram signal based on the target electroencephalogram signal model to obtain a reconstructed signal; and
training the target electroencephalogram signal model based on a difference between the real electroencephalogram signal and the reconstructed signal.

6. An apparatus for training an electroencephalogram signal analysis model, **characterized by** the apparatus comprising:
a second obtaining module (905), configured to obtain random white noise;
a first generation module (906), configured to process the random white noise based on an activation form and an activation location of a source signal to generate a simulated source signal, the source signal representing an electrophysiological signal generated inside the brain of the target object, the activation form representing a frequency range of the source signal, and the activation location representing a location at which the source signal is generated;
a second generation module (907), configured to process the simulated source signal based on a spreading manner of the source signal to generate a simulated electroencephalogram signal;
a first obtaining module (901), configured to obtain a first embedding vector of the simulated source signal and a second embedding vector of the simulated electroencephalogram signal based on an electroencephalogram signal analysis model, the simulated source signal indicating an electrophysiological signal generated inside a brain of a target object, and the simulated electroencephalogram signal indicating an electrical signal measured from a brain scalp of the target object based on a sensor;
a mask module (902), configured to mask the first embedding vector and the second embedding vector respectively to obtain a first mask vector of the simulated source signal and a second mask vector of the simulated electroencephalogram signal, wherein the first mask vector is a vector obtained by covering part of elements in the first embedding vector, and the second mask vector is a vector obtained by covering part of elements in the second embedding vector;
a reconstruction module (903), configured to: perform signal reconstruction on the simulated source signal based on the first mask vector to obtain a reconstructed source signal, and perform signal reconstruction on the simulated electroencephalogram signal based on the second mask vector to obtain a reconstructed electroencephalogram signal, the signal reconstruction comprising encoding and decoding; and
a first training module (904), configured to obtain a training loss of the electroencephalogram signal analysis model based on a difference between the simulated source signal and the reconstructed source signal and a difference between the simulated electroencephalogram signal and the reconstructed electroencephalogram signal, and update a parameter of the electroencephalogram signal analysis model based on the training loss;
wherein the reconstruction module (903) comprises:
an encoding unit (9031), configured to encode the first mask vector and the second mask vector respectively to obtain a first intermediate feature vector of the simulated source signal and a second intermediate feature vector of the simulated electroencephalogram signal;
an alignment unit (9032), configured to perform feature alignment on the first intermediate feature vector and the second intermediate feature vector to obtain a source feature vector of the simulated source signal and an electroencephalogram feature vector of the simulated electroencephalogram signal;
a decoding unit (9033), configured to: fill the source feature vector based on a mask mark of the simulated source signal to obtain a filled source feature vector, the mask mark of the simulated source signal representing a covered element in the embedding vector of the simulated source signal; fill the electroencephalogram feature vector based on a mask mark of the simulated electroencephalogram signal to obtain a filled electroencephalogram feature vector, the mask mark of the simulated electroencephalogram signal representing a covered element in the embedding vector of the simulated electroencephalogram signal; and decode the filled source feature vector and the filled electroencephalogram feature vector respectively to obtain the decoded source feature vector and the decoded electroencephalogram feature vector; and
a mapping unit (9034), configured to perform mapping on the decoded source feature vector to obtain the reconstructed source signal, and performing mapping on the decoded electroencephalogram feature vector to obtain the reconstructed electroencephalogram signal.

7. A computer device, comprising a processor and a memory, the memory being configured to store at least one computer program, the at least one computer program being loaded and executed by the processor to perform the method for training an electroencephalogram signal analysis model according to any one of claims 1 to 5.

8. A computer-readable storage medium, configured to store at least one computer program, the at least one computer program being configured for performing the method for training an electroencephalogram signal analysis model according to any one of claims 1 to 5.

9. A computer program product, comprising a computer program, the computer program implementing, when executed by a processor, the method for training an electroencephalogram signal analysis model according to any one of claims 1 to 5.

## Patentansprüche

1. Verfahren zum Trainieren eines Elektroenzephalogrammsignal-Analysemodells, durchgeführt von einem Server, wobei das Verfahren umfasst:
Erhalten (301) von zufälligem weißem Rauschen;
Verarbeiten (302) des zufälligen weißen Rauschens basierend auf einer Aktivierungsform und einem Aktivierungsort eines Quellsignals, um ein simuliertes Quellsignal zu erzeugen, wobei das Quellsignal ein elektrophysiologisches Signal darstellt, das innerhalb des Gehirns des Zielobjekts erzeugt wird, die Aktivierungsform einen Frequenzbereich des Quellsignals darstellt und der Aktivierungsort einen Ort darstellt, an dem das Quellsignal erzeugt wird;
Verarbeiten (303) des simulierten Quellsignals basierend auf einem Ausbreitungsmodus des Quellsignals, um ein simuliertes Elektroenzephalogrammsignal zu erzeugen;
Erhalten (201, 304) eines ersten Einbettungsvektors des simulierten Quellsignals und eines zweiten Einbettungsvektors des simulierten Elektroenzephalogrammsignals basierend auf einem Elektroenzephalogrammsignal-Analysemodell, wobei das simulierte Quellsignal ein elektrophysiologisches Signal angibt, das innerhalb eines Gehirns eines Zielobjekts erzeugt wird, und das simulierte Elektroenzephalogrammsignal ein elektrisches Signal angibt, das von einer Gehirnkopfhaut des Zielobjekts basierend auf einem Sensor gemessen wird;
Maskieren (202, 305) des ersten Einbettungsvektors und des zweiten Einbettungsvektors jeweils, um einen ersten Maskierungsvektor des simulierten Quellsignals und einen zweiten Maskierungsvektor des simulierten Elektroenzephalogrammsignals zu erhalten, wobei der erste Maskierungsvektor ein Vektor ist, der durch Abdecken eines Teils von Elementen in dem ersten Einbettungsvektor erhalten wird, und der zweite Maskierungsvektor ein Vektor ist, der durch Abdecken eines Teils von Elementen in dem zweiten Einbettungsvektor erhalten wird;
Durchführen (203) von Signalrekonstruktion an dem simulierten Quellsignal basierend auf dem ersten Maskierungsvektor, um ein rekonstruiertes Quellsignal zu erhalten, und Durchführen von Signalrekonstruktion an dem simulierten Elektroenzephalogrammsignal basierend auf dem zweiten Maskierungsvektor, um ein rekonstruiertes Elektroenzephalogrammsignal zu erhalten, wobei die Signalrekonstruktion Kodieren und Dekodieren umfasst, **dadurch gekennzeichnet, dass** das Durchführen (203) umfasst:
Kodieren (306) des ersten Maskierungsvektors und des zweiten Maskierungsvektors jeweils, um einen ersten intermediären Merkmalsvektor des simulierten Quellsignals und einen zweiten intermediären Merkmalsvektor des simulierten Elektroenzephalogrammsignals zu erhalten;
Durchführen (307) von Merkmalsausrichtung an dem ersten intermediären Merkmalsvektor und dem zweiten intermediären Merkmalsvektor, um einen Quell-Merkmalsvektor des simulierten Quellsignals und einen Elektroenzephalogramm-Merkmalsvektor des simulierten Elektroenzephalogrammsignals zu erhalten;
Füllen des Quell-Merkmalsvektors basierend auf einer Maskierungsmarkierung des simulierten Quellsignals, um einen gefüllten Quell-Merkmalsvektor zu erhalten, wobei die Maskierungsmarkierung des simulierten Quellsignals ein abgedecktes Element in dem Einbettungsvektor des simulierten Quellsignals darstellt;
Füllen des Elektroenzephalogramm-Merkmalsvektors basierend auf einer Maskierungsmarkierung des simulierten Elektroenzephalogrammsignals, um einen gefüllten Elektroenzephalogramm-Merkmalsvektor zu erhalten, wobei die Maskierungsmarkierung des simulierten Elektroenzephalogrammsignals ein abgedecktes Element in dem Einbettungsvektor des simulierten Elektroenzephalogrammsignals darstellt; und
Dekodieren des gefüllten Quell-Merkmalsvektors und des gefüllten Elektroenzephalogramm-Merkmalsvektors jeweils, um den dekodierten Quell-Merkmalsvektor und den dekodierten Elektroenzephalogramm-Merkmalsvektor zu erhalten; und
Durchführen von Abbildung (309) an dem dekodierten Quell-Merkmalsvektor, um das rekonstruierte Quellsignal zu erhalten, und Durchführen von Abbildung an dem dekodierten Elektroenzephalogramm-Merkmalsvektor, um das rekonstruierte Elektroenzephalogrammsignal zu erhalten; und
Erhalten eines Trainingsverlusts des Elektroenzephalogrammsignal-Analysemodells basierend auf einer Differenz zwischen dem simulierten Quellsignal und dem rekonstruierten Quellsignal und einer Differenz zwischen dem simulierten Elektroenzephalogrammsignal und dem rekonstruierten Elektroenzephalogrammsignal, und
Aktualisieren eines Parameters des Elektroenzephalogrammsignal-Analysemodells basierend auf dem Trainingsverlust.

2. Verfahren nach Anspruch 1, wobei das Durchführen von Abbildung (309) an dem dekodierten Quell-Merkmalsvektor, um das rekonstruierte Quellsignal zu erhalten, und Durchführen von Abbildung an dem dekodierten Elektroenzephalogramm-Merkmalsvektor, um das rekonstruierte Elektroenzephalogrammsignal zu erhalten, umfasst:
Durchführen von Dekonvolutionsverarbeitung an dem dekodierten Quell-Merkmalsvektor, um einen Dekonvolutions-Quellvektor des simulierten Quellsignals zu erhalten;
Durchführen von Faltungsverarbeitung an dem Dekonvolutions-Quellvektor, um das rekonstruierte Quellsignal zu erhalten;
Durchführen von Dekonvolutionsverarbeitung an dem dekodierten Elektroenzephalogramm-Merkmalsvektor, um einen Dekonvolutions-Elektroenzephalogrammvektor des simulierten Elektroenzephalogrammsignals zu erhalten; und
Durchführen von Faltungsverarbeitung an dem Dekonvolutions-Elektroenzephalogrammvektor, um das rekonstruierte Elektroenzephalogrammsignal zu erhalten.

3. Verfahren nach einem der Ansprüche 1 bis 2, wobei das Erhalten (201, 304) eines ersten Einbettungsvektors eines simulierten Quellsignals und eines zweiten Einbettungsvektors eines simulierten Elektroenzephalogrammsignals umfasst:
Durchführen von zeitlicher Faltung an dem simulierten Quellsignal und dem simulierten Elektroenzephalogrammsignal jeweils, um einen Zeit-Quellvektor des simulierten Quellsignals und einen Zeit-Elektroenzephalogrammvektor des simulierten Elektroenzephalogrammsignals zu erhalten;
Durchführen von Normalisierung an dem Zeit-Quellvektor und dem Zeit-Elektroenzephalogrammvektor jeweils, um einen normalisierten Zeit-Quellvektor und einen normalisierten Zeit-Elektroenzephalogrammvektor zu erhalten; und
Durchführen von räumlicher Faltung an dem normalisierten Zeit-Quellvektor und dem normalisierten Zeit-Elektroenzephalogrammvektor jeweils, um den ersten Einbettungsvektor des simulierten Quellsignals und den zweiten Einbettungsvektor des simulierten Elektroenzephalogrammsignals zu erhalten.

4. Verfahren nach einem der Ansprüche 1 bis 2, wobei das Erhalten eines Trainingsverlusts des Elektroenzephalogrammsignal-Analysemodells basierend auf einer Differenz zwischen dem simulierten Quellsignal und dem rekonstruierten Quellsignal und einer Differenz zwischen dem simulierten Elektroenzephalogrammsignal und dem rekonstruierten Elektroenzephalogrammsignal umfasst:
Erhalten eines Rekonstruktionsverlusts des simulierten Quellsignals basierend auf der Differenz zwischen dem simulierten Quellsignal und dem rekonstruierten Quellsignal;
Erhalten eines Rekonstruktionsverlusts des simulierten Elektroenzephalogrammsignals basierend auf der Differenz zwischen dem simulierten Elektroenzephalogrammsignal und dem rekonstruierten Elektroenzephalogrammsignal; und
Durchführen von gewichteter Summierung an dem Rekonstruktionsverlust des simulierten Quellsignals und dem Rekonstruktionsverlust des simulierten Elektroenzephalogrammsignals, um den Trainingsverlust des Elektroenzephalogrammsignal-Analysemodells zu erhalten.

5. Verfahren nach einem der Ansprüche 1 bis 2, wobei das Elektroenzephalogrammsignal-Analysemodell ein Ziel-Einbettungsmodul und einen ersten Enkoder umfasst, das Ziel-Einbettungsmodul konfiguriert ist, um Einbettungsverarbeitung an dem simulierten Elektroenzephalogrammsignal durchzuführen, und der erste Enkoder konfiguriert ist, um den Einbettungsvektor des simulierten Elektroenzephalogrammsignals zu kodieren; und
das Verfahren ferner umfasst:
Konstruieren eines Ziel-Elektroenzephalogrammsignal-Modells basierend auf dem Ziel-Einbettungsmodul, dem ersten Enkoder und einem ersten Dekoder, wobei der erste Dekoder konfiguriert ist, um einen Elektroenzephalogramm-Merkmalsvektor eines realen Elektroenzephalogrammsignals zu dekodieren, das Ziel-Elektroenzephalogrammsignal-Modell Signalrekonstruktion an dem Elektroenzephalogrammsignal durchführt und das reale Elektroenzephalogrammsignal das elektrische Signal darstellt, das von der Gehirnkopfhaut des Zielobjekts basierend auf dem Sensor gemessen wird;
Durchführen von Signalrekonstruktion an dem realen Elektroenzephalogrammsignal basierend auf dem Ziel-Elektroenzephalogrammsignal-Modell, um ein rekonstruiertes Signal zu erhalten; und
Trainieren des Ziel-Elektroenzephalogrammsignal-Modells basierend auf einer Differenz zwischen dem realen Elektroenzephalogrammsignal und dem rekonstruierten Signal.

6. Vorrichtung zum Trainieren eines Elektroenzephalogrammsignal-Analysemodells, **gekennzeichnet durch** die Vorrichtung umfassend:
ein zweites Erhaltungsmodul (905), das konfiguriert ist, um zufälliges weißes Rauschen zu erhalten;
ein erstes Erzeugungsmodul (906), das konfiguriert ist, um das zufällige weiße Rauschen basierend auf einer Aktivierungsform und einem Aktivierungsort eines Quellsignals zu verarbeiten, um ein simuliertes Quellsignal zu erzeugen, wobei das Quellsignal ein elektrophysiologisches Signal darstellt, das innerhalb des Gehirns des Zielobjekts erzeugt wird, die Aktivierungsform einen Frequenzbereich des Quellsignals darstellt und der Aktivierungsort einen Ort darstellt, an dem das Quellsignal erzeugt wird;
ein zweites Erzeugungsmodul (907), das konfiguriert ist, um das simulierte Quellsignal basierend auf einer Ausbreitungsweise des Quellsignals zu verarbeiten, um ein simuliertes Elektroenzephalogrammsignal zu erzeugen;
ein erstes Erhaltungsmodul (901), das konfiguriert ist, um einen ersten Einbettungsvektor des simulierten Quellsignals und einen zweiten Einbettungsvektor des simulierten Elektroenzephalogrammsignals basierend auf einem Elektroenzephalogrammsignal-Analysemodell zu erhalten, wobei das simulierte Quellsignal ein elektrophysiologisches Signal angibt, das innerhalb eines Gehirns eines Zielobjekts erzeugt wird, und das simulierte Elektroenzephalogrammsignal ein elektrisches Signal angibt, das von einer Gehirnkopfhaut des Zielobjekts basierend auf einem Sensor gemessen wird;
ein Maskierungsmodul (902), das konfiguriert ist, um den ersten Einbettungsvektor und den zweiten Einbettungsvektor jeweils zu maskieren, um einen ersten Maskierungsvektor des simulierten Quellsignals und einen zweiten Maskierungsvektor des simulierten Elektroenzephalogrammsignals zu erhalten, wobei der erste Maskierungsvektor ein Vektor ist, der durch Abdecken eines Teils von Elementen in dem ersten Einbettungsvektor erhalten wird, und der zweite Maskierungsvektor ein Vektor ist, der durch Abdecken eines Teils von Elementen in dem zweiten Einbettungsvektor erhalten wird;
ein Rekonstruktionsmodul (903), das konfiguriert ist zum:
Durchführen von Signalrekonstruktion an dem simulierten Quellsignal basierend auf dem ersten Maskierungsvektor, um ein rekonstruiertes Quellsignal zu erhalten, und Durchführen von Signalrekonstruktion an dem simulierten Elektroenzephalogrammsignal basierend auf dem zweiten Maskierungsvektor, um ein rekonstruiertes Elektroenzephalogrammsignal zu erhalten, wobei die Signalrekonstruktion Kodieren und Dekodieren umfasst; und
ein erstes Trainingsmodul (904), das konfiguriert ist, um einen Trainingsverlust des Elektroenzephalogrammsignal-Analysemodells basierend auf einer Differenz zwischen dem simulierten Quellsignal und dem rekonstruierten Quellsignal und einer Differenz zwischen dem simulierten Elektroenzephalogrammsignal und dem rekonstruierten Elektroenzephalogrammsignal zu erhalten, und einen Parameter des Elektroenzephalogrammsignal-Analysemodells basierend auf dem Trainingsverlust zu aktualisieren;
wobei das Rekonstruktionsmodul (903) umfasst:
eine Kodiereinheit (9031), die konfiguriert ist, um den ersten Maskierungsvektor und den zweiten Maskierungsvektor jeweils zu kodieren, um einen ersten intermediären Merkmalsvektor des simulierten Quellsignals und einen zweiten intermediären Merkmalsvektor des simulierten Elektroenzephalogrammsignals zu erhalten;
eine Ausrichtungseinheit (9032), die konfiguriert ist, um Merkmalsausrichtung an dem ersten intermediären Merkmalsvektor und dem zweiten intermediären Merkmalsvektor durchzuführen, um einen Quell-Merkmalsvektor des simulierten Quellsignals und einen Elektroenzephalogramm-Merkmalsvektor des simulierten Elektroenzephalogrammsignals zu erhalten;
eine Dekodiereinheit (9033), die konfiguriert ist zum: Füllen des Quell-Merkmalsvektors basierend auf einer Maskierungsmarkierung des simulierten Quellsignals, um einen gefüllten Quell-Merkmalsvektor zu erhalten, wobei die Maskierungsmarkierung des simulierten Quellsignals ein abgedecktes Element in dem Einbettungsvektor des simulierten Quellsignals darstellt; Füllen des Elektroenzephalogramm-Merkmalsvektors basierend auf einer Maskierungsmarkierung des simulierten Elektroenzephalogrammsignals, um einen gefüllten Elektroenzephalogramm-Merkmalsvektor zu erhalten, wobei die Maskierungsmarkierung des simulierten Elektroenzephalogrammsignals ein abgedecktes Element in dem Einbettungsvektor des simulierten Elektroenzephalogrammsignals darstellt; und Dekodieren des gefüllten Quell-Merkmalsvektors und des gefüllten Elektroenzephalogramm-Merkmalsvektors jeweils, um den dekodierten Quell-Merkmalsvektor und den dekodierten Elektroenzephalogramm-Merkmalsvektor zu erhalten; und
eine Abbildungseinheit (9034), die konfiguriert ist, um Abbildung an dem dekodierten Quell-Merkmalsvektor durchzuführen, um das rekonstruierte Quellsignal zu erhalten, und Abbildung an dem dekodierten Elektroenzephalogramm-Merkmalsvektor durchzuführen, um das rekonstruierte Elektroenzephalogrammsignal zu erhalten.

7. Computergerät, umfassend einen Prozessor und einen Speicher, wobei der Speicher konfiguriert ist, um mindestens ein Computerprogramm zu speichern, wobei das mindestens eine Computerprogramm von dem Prozessor geladen und ausgeführt wird, um das Verfahren zum Trainieren eines Elektroenzephalogrammsignal-Analysemodells nach einem der Ansprüche 1 bis 5 durchzuführen.

8. Computerlesbares Speichermedium, das konfiguriert ist, um mindestens ein Computerprogramm zu speichern, wobei das mindestens eine Computerprogramm zum Durchführen des Verfahrens zum Trainieren eines Elektroenzephalogrammsignal-Analysemodells nach einem der Ansprüche 1 bis 5 konfiguriert ist.

9. Computerprogrammprodukt, umfassend ein Computerprogramm, wobei das Computerprogramm, wenn es von einem Prozessor ausgeführt wird, das Verfahren zum Trainieren eines Elektroenzephalogrammsignal-Analysemodells nach einem der Ansprüche 1 bis 5 implementiert.

## Revendications

1. Procédé d'entraînement d'un modèle d'analyse de signal électroencéphalographique, exécuté par un serveur, le procédé comprenant :
l'obtention (301) d'un bruit blanc aléatoire ;
le traitement (302) du bruit blanc aléatoire sur la base d'une forme d'activation et d'un emplacement d'activation d'un signal source pour générer un signal source simulé, le signal source représentant un signal électrophysiologique généré à l'intérieur du cerveau de l'objet cible, la forme d'activation représentant une plage de fréquences du signal source, et l'emplacement d'activation représentant un emplacement auquel le signal source est généré ;
le traitement (303) du signal source simulé sur la base d'un mode de propagation du signal source pour générer un signal électroencéphalographique simulé ;
l'obtention (201, 304) d'un premier vecteur d'incorporation du signal source simulé et d'un deuxième vecteur d'incorporation du signal électroencéphalographique simulé sur la base d'un modèle d'analyse de signal électroencéphalographique, le signal source simulé indiquant un signal électrophysiologique généré à l'intérieur d'un cerveau d'un objet cible, et le signal électroencéphalographique simulé indiquant un signal électrique mesuré à partir d'un cuir chevelu de l'objet cible sur la base d'un capteur ;
le masquage (202, 305) du premier vecteur d'incorporation et du deuxième vecteur d'incorporation respectivement pour obtenir un premier vecteur de masque du signal source simulé et un deuxième vecteur de masque du signal électroencéphalographique simulé, dans lequel le premier vecteur de masque est un vecteur obtenu en couvrant une partie des éléments dans le premier vecteur d'incorporation, et le deuxième vecteur de masque est un vecteur obtenu en couvrant une partie des éléments dans le deuxième vecteur d'incorporation ;
la réalisation (203) d'une reconstruction de signal sur le signal source simulé sur la base du premier vecteur de masque pour obtenir un signal source reconstruit, et la réalisation d'une reconstruction de signal sur le signal électroencéphalographique simulé sur la base du deuxième vecteur de masque pour obtenir un signal électroencéphalographique reconstruit, la reconstruction de signal comprenant un encodage et un décodage, **caractérisé en ce que** la réalisation (203) comprend :
l'encodage (306) du premier vecteur de masque et du deuxième vecteur de masque respectivement pour obtenir un premier vecteur de caractéristiques intermédiaire du signal source simulé et un deuxième vecteur de caractéristiques intermédiaire du signal électroencéphalographique simulé ;
la réalisation (307) d'un alignement de caractéristiques sur le premier vecteur de caractéristiques intermédiaire et le deuxième vecteur de caractéristiques intermédiaire pour obtenir un vecteur de caractéristiques source du signal source simulé et un vecteur de caractéristiques électroencéphalographique du signal électroencéphalographique simulé ;
le remplissage du vecteur de caractéristiques source sur la base d'une marque de masque du signal source simulé pour obtenir un vecteur de caractéristiques source rempli, la marque de masque du signal source simulé représentant un élément couvert dans le vecteur d'incorporation du signal source simulé ;
le remplissage du vecteur de caractéristiques électroencéphalographique sur la base d'une marque de masque du signal électroencéphalographique simulé pour obtenir un vecteur de caractéristiques électroencéphalographique rempli, la marque de masque du signal électroencéphalographique simulé représentant un élément couvert dans le vecteur d'incorporation du signal électroencéphalographique simulé ; et
le décodage du vecteur de caractéristiques source rempli et du vecteur de caractéristiques électroencéphalographique rempli respectivement pour obtenir le vecteur de caractéristiques source décodé et le vecteur de caractéristiques électroencéphalographique décodé ; et
la réalisation d'un mappage (309) sur le vecteur de caractéristiques source décodé pour obtenir le signal source reconstruit, et la réalisation d'un mappage sur le vecteur de caractéristiques électroencéphalographique décodé pour obtenir le signal électroencéphalographique reconstruit ; et
l'obtention d'une perte d'entraînement du modèle d'analyse de signal électroencéphalographique sur la base d'une différence entre le signal source simulé et le signal source reconstruit et d'une différence entre le signal électroencéphalographique simulé et le signal électroencéphalographique reconstruit, et la mise à jour d'un paramètre du modèle d'analyse de signal électroencéphalographique sur la base de la perte d'entraînement.

2. Procédé selon la revendication 1, dans lequel la réalisation du mappage (309) sur le vecteur de caractéristiques source décodé pour obtenir le signal source reconstruit, et la réalisation du mappage sur le vecteur de caractéristiques électroencéphalographique décodé pour obtenir le signal électroencéphalographique reconstruit comprend :
la réalisation d'un traitement de déconvolution sur le vecteur de caractéristiques source décodé pour obtenir un vecteur source de déconvolution du signal source simulé ;
la réalisation d'un traitement de convolution sur le vecteur source de déconvolution pour obtenir le signal source reconstruit ;
la réalisation d'un traitement de déconvolution sur le vecteur de caractéristiques électroencéphalographique décodé pour obtenir un vecteur électroencéphalographique de déconvolution du signal électroencéphalographique simulé ; et
la réalisation d'un traitement de convolution sur le vecteur électroencéphalographique de déconvolution pour obtenir le signal électroencéphalographique reconstruit.

3. Procédé selon l'une quelconque des revendications 1 à 2, dans lequel l'obtention (201, 304) d'un premier vecteur d'incorporation d'un signal source simulé et d'un deuxième vecteur d'incorporation d'un signal électroencéphalographique simulé comprend :
la réalisation d'une convolution temporelle sur le signal source simulé et le signal électroencéphalographique simulé respectivement pour obtenir un vecteur source temporel du signal source simulé et un vecteur électroencéphalographique temporel du signal électroencéphalographique simulé ;
la réalisation d'une normalisation sur le vecteur source temporel et le vecteur électroencéphalographique temporel respectivement pour obtenir un vecteur source temporel normalisé et un vecteur électroencéphalographique temporel normalisé ; et
la réalisation d'une convolution spatiale sur le vecteur source temporel normalisé et le vecteur électroencéphalographique temporel normalisé respectivement pour obtenir le premier vecteur d'incorporation du signal source simulé et le deuxième vecteur d'incorporation du signal électroencéphalographique simulé.

4. Procédé selon l'une quelconque des revendications 1 à 2, dans lequel l'obtention d'une perte d'entraînement du modèle d'analyse de signal électroencéphalographique sur la base d'une différence entre le signal source simulé et le signal source reconstruit et d'une différence entre le signal électroencéphalographique simulé et le signal électroencéphalographique reconstruit comprend :
l'obtention d'une perte de reconstruction du signal source simulé sur la base de la différence entre le signal source simulé et le signal source reconstruit ;
l'obtention d'une perte de reconstruction du signal électroencéphalographique simulé sur la base de la différence entre le signal électroencéphalographique simulé et le signal électroencéphalographique reconstruit ; et
la réalisation d'une sommation pondérée sur la perte de reconstruction du signal source simulé et la perte de reconstruction du signal électroencéphalographique simulé pour obtenir la perte d'entraînement du modèle d'analyse de signal électroencéphalographique.

5. Procédé selon l'une quelconque des revendications 1 à 2, dans lequel le modèle d'analyse de signal électroencéphalographique comprend un module d'incorporation cible et un premier encodeur, le module d'incorporation cible est configuré pour réaliser un traitement d'incorporation sur le signal électroencéphalographique simulé, et le premier encodeur est configuré pour encoder le vecteur d'incorporation du signal électroencéphalographique simulé ; et
le procédé comprend en outre :
la construction d'un modèle de signal électroencéphalographique cible sur la base du module d'incorporation cible, du premier encodeur et d'un premier décodeur, le premier décodeur étant configuré pour décoder un vecteur de caractéristiques électroencéphalographique d'un signal électroencéphalographique réel, le modèle de signal électroencéphalographique cible réalisant une reconstruction de signal sur le signal électroencéphalographique, et le signal électroencéphalographique réel représentant le signal électrique mesuré à partir du cuir chevelu de l'objet cible sur la base du capteur ;
la réalisation d'une reconstruction de signal sur le signal électroencéphalographique réel sur la base du modèle de signal électroencéphalographique cible pour obtenir un signal reconstruit ; et
l'entraînement du modèle de signal électroencéphalographique cible sur la base d'une différence entre le signal électroencéphalographique réel et le signal reconstruit.

6. Appareil d'entraînement d'un modèle d'analyse de signal électroencéphalographique, **caractérisé en ce que** l'appareil comprend :
un deuxième module d'obtention (905), configuré pour obtenir un bruit blanc aléatoire ;
un premier module de génération (906), configuré pour traiter le bruit blanc aléatoire sur la base d'une forme d'activation et d'un emplacement d'activation d'un signal source pour générer un signal source simulé, le signal source représentant un signal électrophysiologique généré à l'intérieur du cerveau de l'objet cible, la forme d'activation représentant une plage de fréquences du signal source, et l'emplacement d'activation représentant un emplacement auquel le signal source est généré ;
un deuxième module de génération (907), configuré pour traiter le signal source simulé sur la base d'un mode de propagation du signal source pour générer un signal électroencéphalographique simulé ;
un premier module d'obtention (901), configuré pour obtenir un premier vecteur d'incorporation du signal source simulé et un deuxième vecteur d'incorporation du signal électroencéphalographique simulé sur la base d'un modèle d'analyse de signal électroencéphalographique, le signal source simulé indiquant un signal électrophysiologique généré à l'intérieur d'un cerveau d'un objet cible, et le signal électroencéphalographique simulé indiquant un signal électrique mesuré à partir d'un cuir chevelu de l'objet cible sur la base d'un capteur ;
un module de masque (902), configuré pour masquer le premier vecteur d'incorporation et le deuxième vecteur d'incorporation respectivement pour obtenir un premier vecteur de masque du signal source simulé et un deuxième vecteur de masque du signal électroencéphalographique simulé, dans lequel le premier vecteur de masque est un vecteur obtenu en couvrant une partie des éléments dans le premier vecteur d'incorporation, et le deuxième vecteur de masque est un vecteur obtenu en couvrant une partie des éléments dans le deuxième vecteur d'incorporation ;
un module de reconstruction (903), configuré pour : réaliser une reconstruction de signal sur le signal source simulé sur la base du premier vecteur de masque pour obtenir un signal source reconstruit, et réaliser une reconstruction de signal sur le signal électroencéphalographique simulé sur la base du deuxième vecteur de masque pour obtenir un signal électroencéphalographique reconstruit, la reconstruction de signal comprenant un encodage et un décodage ; et
un premier module d'entraînement (904), configuré pour obtenir une perte d'entraînement du modèle d'analyse de signal électroencéphalographique sur la base d'une différence entre le signal source simulé et le signal source reconstruit et d'une différence entre le signal électroencéphalographique simulé et le signal électroencéphalographique reconstruit, et mettre à jour un paramètre du modèle d'analyse de signal électroencéphalographique sur la base de la perte d'entraînement ;
dans lequel le module de reconstruction (903) comprend :
une unité d'encodage (9031), configurée pour encoder le premier vecteur de masque et le deuxième vecteur de masque respectivement pour obtenir un premier vecteur de caractéristiques intermédiaire du signal source simulé et un deuxième vecteur de caractéristiques intermédiaire du signal électroencéphalographique simulé ;
une unité d'alignement (9032), configurée pour réaliser un alignement de caractéristiques sur le premier vecteur de caractéristiques intermédiaire et le deuxième vecteur de caractéristiques intermédiaire pour obtenir un vecteur de caractéristiques source du signal source simulé et un vecteur de caractéristiques électroencéphalographique du signal électroencéphalographique simulé ;
une unité de décodage (9033), configurée pour : remplir le vecteur de caractéristiques source sur la base d'une marque de masque du signal source simulé pour obtenir un vecteur de caractéristiques source rempli, la marque de masque du signal source simulé représentant un élément couvert dans le vecteur d'incorporation du signal source simulé ; remplir le vecteur de caractéristiques électroencéphalographique sur la base d'une marque de masque du signal électroencéphalographique simulé pour obtenir un vecteur de caractéristiques électroencéphalographique rempli, la marque de masque du signal électroencéphalographique simulé représentant un élément couvert dans le vecteur d'incorporation du signal électroencéphalographique simulé ; et décoder le vecteur de caractéristiques source rempli et le vecteur de caractéristiques électroencéphalographique rempli respectivement pour obtenir le vecteur de caractéristiques source décodé et le vecteur de caractéristiques électroencéphalographique décodé ; et
une unité de mappage (9034), configurée pour réaliser un mappage sur le vecteur de caractéristiques source décodé pour obtenir le signal source reconstruit, et réaliser un mappage sur le vecteur de caractéristiques électroencéphalographique décodé pour obtenir le signal électroencéphalographique reconstruit.

7. Dispositif informatique, comprenant un processeur et une mémoire, la mémoire étant configurée pour stocker au moins un programme informatique, l'au moins un programme informatique étant chargé et exécuté par le processeur pour exécuter le procédé d'entraînement d'un modèle d'analyse de signal électroencéphalographique selon l'une quelconque des revendications 1 à 5.

8. Support de stockage lisible par ordinateur, configuré pour stocker au moins un programme informatique, l'au moins un programme informatique étant configuré pour exécuter le procédé d'entraînement d'un modèle d'analyse de signal électroencéphalographique selon l'une quelconque des revendications 1 à 5.

9. Produit programme d'ordinateur, comprenant un programme informatique, le programme informatique mettant en œuvre, lorsqu'il est exécuté par un processeur, le procédé d'entraînement d'un modèle d'analyse de signal électroencéphalographique selon l'une quelconque des revendications 1 à 5.
